# EUROPEAN PATENT APPLICATION

(11) **EP 4 269 447 A1**
(43) Date of publication of application: **01.11.2023**
(21) Application number: 21909437.2
(22) Date of filing: 22.12.2021
(51) Int. Cl.: C07K 19/00, C07K 16/30, C12N 15/62, A61P 35/00

(54) **COMPLEX OF ANTI-IL-4R ANTIBODY OR ANTIGEN-BINDING FRAGMENT THEREOF AND MEDICAL USE THEREOF**

(30) Priority: 22.12.2020 CN 202011529235
(71) Applicant: Jiangsu Hengrui Pharmaceuticals Co., Ltd., Lianyungang, Jiangsu 222047 (CN); Shanghai Shengdi Pharmaceutical Co., Ltd, Shanghai 201210 (CN)
(72) Inventor: WANG, Huan, Shanghai 201210 (CN); LIN, Yuan, Shanghai 201210 (CN); TANG, Yucheng, Shanghai 201210 (CN); KE, Ke, Shanghai 201210 (CN); LIN, Kan, Shanghai 201210 (CN); LIAO, Cheng, Shanghai 201210 (CN)
(74) Representative: Regimbeau
(86) International application number: PCT/CN2021/140310
(87) International publication number: WO 2022/135441

(57) **Abstract**

Provided are a complex of an anti-IL-4R antibody or an antigen-binding fragment thereof, and a medical use thereof. Specifically, provided are a complex of an antibody that specifically binds to IL-4R or an antigen-binding fragment thereof covalently linked to a toxin, a pharmaceutical composition comprising the complex, and a use thereof in the preparation of a drug for treating IL-4R-mediated diseases or disorders, especially a use in the preparation of an anti-cancer drug.

## Description

The present application claims priority to Chinese Patent Application No. 202011529235.1 filed on Dec. 22, 2020.

### TECHNICAL FIELD

The present application relates to a complex of an anti-IL-4R antibody or an antigen-binding fragment thereof, and use thereof as an anti-cancer drug.

### BACKGROUND

Malignant tumors (cancer) are second only to heart disease as the cause of death in the world. Glioblastomas (GBMs) are the most common malignant primary brain tumors. The median survival of glioblastoma patients undergoing standard therapy is less than 15 months. Despite advances in tumor biology, they have not been converted into products or therapies that benefit patients. Therefore, there is an urgent need to develop an effective method for treating glioblastomas. IL-4R, interleukin 4 receptor, is expressed on glioblastoma cells and infiltrating immunosuppressive cells (e.g., tumor-associated macrophages (TAMs) and myeloid-derived suppressor cells (MDSCs)). IL-4R-targeted therapy can not only kill tumor cells but also improve the tumor microenvironment. IL-4R-targeted therapy shows better efficacy than traditional therapy in both the preclinical animal model of glioblastomas and the early clinical trials.

MDNA55, developed by Medicenna Inc., is a fusion protein formed with IL-4 and the bacterial toxin pseudomonas exotoxin A (PE38KDEL). It has shown positive effect in clinical trials during development (see WO9527732; Biochem. J. (1995) 307,29-37; and CANCER RESEARCH 55,3357-3363, August 1, 1995).

However, there are problems with first-generation immunotoxin therapy, such as insufficient affinity for the target. One of the major and difficult points in the research of immunotoxin therapy for tumors is to improve the targeting ability and affinity of immunotoxins (see "China Tumor Clinical Yearbook 2009", Ed. Zhao Ping, Peking Union Medical College Press, Jul. 2010, p. 61).

Antibody-drug conjugates (ADCs) are intended to combine the selectivity of monoclonal antibodies (mAbs) with the cytotoxic potential of chemotherapeutic drugs. An antibody-drug conjugate mainly consists of three components: "antibody", "linker" and "drug". Theoretically, antibody-drug conjugates are more effective than traditional fully or partially humanized antibodies or antibody fragments due to the capability of releasing highly active cytotoxins in tumor tissues. Antibody-drug conjugates are more tolerable and have fewer side effects than fusion proteins. The affinity of antibody-drug conjugates for the target antigen affects the process of antibody-drug conjugates permeating into tumor tissues to bind to the target antigen and directly affects the binding efficiency of antibody-drug conjugates (see "Biotechnological Pharmaceutics", Ed. Feng Meiqing, China Medical Science Press, Jan. 2016, pp. 209-211).

One of the most crucial factors in the design of an antibody-drug conjugate is the choice of the antibody; it is important that its specificity to the antigen is high. Antibodies that are lacking in high specificity and that cross-react with other antigens may fail to produce their desired effect; for example, they interact with healthy tissues to cause off-target toxicity, or they are cleared from the body too soon before reaching tumors (see: "Introduction to Antibody-Drug Conjugates (ADCs)", Ilona Pysz, Paul J. M. Jackson and David E. Thurston, CHAPTER 1: Introduction to Antibody-Drug Conjugates (ADCs), in Cytotoxic Payloads for Antibody-Drug Conjugates, 2019).

There have now been a number of pharmaceutical companies in various countries that are developing monoclonal antibodies against IL-4R; related patent applications include, for example, WO2010053751, WO2001092340, WO2008054606 and WO2014031610. The inventors' prior application WO2020038454 also relates to a class of novel anti-IL-4R antibodies. However, IL-4R-directed antibody-drug conjugates are rarely explicitly reported; for example, WO2015188934, WO2014124227, WO2018217227, etc. all merely mention in a general way that the antibodies in antibody-drug conjugates optionally target IL-4R, but none discloses a concrete anti-IL-4R antibody-drug conjugate.

According to the present disclosure, a second-generation immunotoxin drug that delivers a drug to a target is prepared with a high-affinity anti-IL-4R antibody for the purpose of intensively killing cancer cells and fulfilling the need to treat cancer or delay the progression of cancer.

### SUMMARY

The present disclosure relates to a complex of an anti-IL-4R antibody or an antigen-binding fragment thereof, a preparation method therefor and use thereof in pharmaceutics.

### Complex

The present disclosure provides a complex of an anti-IL-4R antibody or an antigen-binding fragment thereof, the complex comprising:
the anti-IL-4R antibody or the antigen-binding fragment thereof, and one or more toxin molecules.

In some embodiments, the anti-IL-4R antibody or the antigen-binding fragment thereof in the complex comprises a heavy chain variable region and a light chain variable region, wherein the heavy chain variable region comprises:
(I) a HCDR1, a HCDR2 and a HCDR3 whose amino acid sequences are set forth in SEQ ID NO: 3, SEQ ID NO: 4 and SEQ ID NO: 5, respectively; or
(II) a HCDR1, a HCDR2 and a HCDR3 whose amino acid sequences are set forth in SEQ ID NO: 11, SEQ ID NO: 12 and SEQ ID NO: 13, respectively;
and/or, the light chain variable region of the antibody comprises:
(I) a LCDR1, a LCDR2 and a LCDR3 whose amino acid sequences are set forth in SEQ ID NO: 6, SEQ ID NO: 7 and SEQ ID NO: 8, respectively; or
(II) a LCDR1, a LCDR2 and a LCDR3 whose amino acid sequences are set forth in SEQ ID NO: 14, SEQ ID NO: 15 and SEQ ID NO: 16, respectively; or
(III) a LCDR1, a LCDR2 and a LCDR3 whose amino acid sequences are set forth in SEQ ID NO: 38, SEQ ID NO: 7 and SEQ ID NO: 40, respectively; or
(IV) a LCDR1, a LCDR2 and a LCDR3 whose amino acid sequences are set forth in SEQ ID NO: 42, SEQ ID NO: 39 and SEQ ID NO: 8, respectively;
wherein the anti-IL-4R antibody or the antigen-binding fragment thereof is covalently or non-covalently linked to the toxin.

**Table 1. The CDR sequences of the anti-IL-4R antibody or the antigen-binding fragment thereof**

| Name | Sequence | No. |
|---|---|---|
| HCDR1 | GFTFSDYGMH | SEQ ID NO: 3 |
| HCDR2 | FISSGSSIIYYADIVKG | SEQ ID NO: 4 |
| HCDR3 | GNKRGFFDY | SEQ ID NO: 5 |
| LCDR1 | NASSSVSYMY | SEQ ID NO: 6 |
| LCDR2 | LTSNLAS | SEQ ID NO: 7 |
| LCDR3 | QQWRSNPPMLT | SEQ ID NO: 8 |
| HCDR1 | GYTFTSYWMH | SEQ ID NO: 11 |
| HCDR2 | LIHPNSDTTKFSENFKT | SEQ ID NO: 12 |
| HCDR3 | SKIITTIVARHWYFDV | SEQ ID NO: 13 |
| LCDR1 | KASQSVDYGGDSYMN | SEQ ID NO: 14 |
| LCDR2 | AASNLES | SEQ ID NO: 15 |
| LCDR3 | QHSNENPPT | SEQ ID NO: 16 |
| LCDR1 | RASSSVPYMY | SEQ ID NO: 38 |
| LCDR2 | LASSRPS | SEQ ID NO: 39 |
| LCDR3 | QQWRAYPPMLT | SEQ ID NO: 40 |
| LCDR1 | RASPGVPPLA | SEQ ID NO: 42 |

In some embodiments, the anti-IL-4R antibody or the antigen-binding fragment thereof comprises any one selected from the group consisting of (I) to (IV):
(I) a heavy chain variable region comprising a HCDR1, a HCDR2 and a HCDR3 whose amino acid sequences are set forth in SEQ ID NO: 3, SEQ ID NO: 4 and SEQ ID NO: 5, respectively; and
   a light chain variable region comprising a LCDR1, a LCDR2 and a LCDR3 whose amino acid sequences are set forth in SEQ ID NO: 6, SEQ ID NO: 7 and SEQ ID NO: 8, respectively;
(II) a heavy chain variable region comprising a HCDR1, a HCDR2 and a HCDR3 whose amino acid sequences are set forth in SEQ ID NO: 11, SEQ ID NO: 12 and SEQ ID NO: 13, respectively; and
   a light chain variable region comprising a LCDR1, a LCDR2 and a LCDR3 whose amino acid sequences are set forth in SEQ ID NO: 14, SEQ ID NO: 15 and SEQ ID NO: 16, respectively;
(III) a heavy chain variable region comprising a HCDR1, a HCDR2 and a HCDR3 whose amino acid sequences are set forth in SEQ ID NO: 3, SEQ ID NO: 4 and SEQ ID NO: 5, respectively; and
   a light chain variable region comprising a LCDR1, a LCDR2 and a LCDR3 whose amino acid sequences are set forth in SEQ ID NO: 38, SEQ ID NO: 7 and SEQ ID NO: 40, respectively;
(IV) a heavy chain variable region comprising a HCDR1, a HCDR2 and a HCDR3 whose amino acid sequences are set forth in SEQ ID NO: 3, SEQ ID NO: 4 and SEQ ID NO: 5, respectively; and
a light chain variable region comprising a LCDR1, a LCDR2 and a LCDR3 whose amino acid sequences are set forth in SEQ ID NO: 42, SEQ ID NO: 39 and SEQ ID NO: 8, respectively.

In some embodiments, the anti-IL-4R antibody or the antigen-binding fragment thereof comprises a heavy chain variable region and a light chain variable region, wherein:
the heavy chain variable region comprises:
   (I) the sequence set forth in SEQ ID NO: 1 or a sequence having at least 70%, 80%, 90%, 95%, 98% or 99% identity to SEQ ID NO: 1; or
   (II) the sequence set forth in SEQ ID NO: 9 or a sequence having at least 70%, 80%, 90%, 95%, 98% or 99% identity to SEQ ID NO: 9; or
   (III) the sequence set forth in SEQ ID NO: 43 or a sequence having at least 70%, 80%, 90%, 95%, 98% or 99% identity to SEQ ID NO: 43; or
and/or, the light chain variable region comprises:
   (I) the sequence set forth in SEQ ID NO: 2 or a sequence having at least 70%, 80%, 90%, 95%, 98% or 99% identity to SEQ ID NO: 2; or
   (II) the sequence set forth in SEQ ID NO: 10 or a sequence having at least 70%, 80%, 90%, 95%, 98% or 99% identity to SEQ ID NO: 10; or
   (III) the sequence set forth in SEQ ID NO: 37 or a sequence having at least 70%, 80%, 90%, 95%, 98% or 99% identity to SEQ ID NO: 37; or
   (IV) the sequence set forth in SEQ ID NO: 41 or a sequence having at least 70%, 80%, 90%, 95%, 98% or 99% identity to SEQ ID NO: 41.
25G7 HCVR (heavy chain variable region of 25G7) 
EVQLVESGGGLVKPGGSLKLSCAASGFTFSDYGMHWVRQAPEKGLEWVAFISS
GSSIIYYADIVKGRSTISRDNAKNTLFLQMTSLRSEDTAMYYCTRGNKRGFFDY
WGQGTILTVSS
SEQ ID NO: 1;
25G7 LCVR (light chain variable region of 25G7) 
QIVLTQSPALMSASPGEKVTMTCNASSSVSYMYWYQRKPRSSPKPWIYLTSNLA
SGVPVRFSGSGSGTSYSLTISSMEAEDAATYYCQQWRSNPPMLTFGSGTKLEVK
SEQ ID NO: 2;
7B10 HCVR (heavy chain variable region of 7B10) 
QVQLQQPGTELLKPGASVSLSCKASGYTFTSYWMHWVKQRPGQGLEWIGLIHP
NSDTTKFSENFKTRATLTIDKSSSTAYMKLSSLTSEDSAVYYCAKSKIITTIVARH
WYFDVWGTGTTVTVSS
SEQID NO: 9;
7B10 LCVR (light chain variable region of 7B10) 
DIVLTQSPPSLAVSLGQRATISCKASQSVDYGGDSYMNWYQQKLGQPPKVLIYA
ASNLESGIPARFSGSGSGTDFTLNIHPVEEEDVATYYCQHSNENPPTFGGGTKLEI
K
SEQID NO: 10;
hu25G7-ALCVR (light chain variable region of hu25G7-A) 
EIVLTQSPATLSLSPGERATLSCRASSSVPYMYWYQQKPGQAPRLLIYLTSNLAS
GIPARFSGSGSGTDFTLTISSLEPEDFAVYYCQQWRAYPPMLTFGGGTKVEIK
SEQ ID NO: 37;
hu25G7-B LCVR (light chain variable region of hu25G7-B) 
EIVLTQSPATLSLSPGERATLSCRASPGVPPLAWYQQKPGQAPRLLIYLASSRPSGI
PARFSGSGSGTDFTLTISSLEPEDFAVYYCQQWRSNPPMLTFGGGTKVEIK
SEQ ID NO: 41
hu25G7-VH (heavy chain variable region of hu25G7)

EVQLVESGGGLVQPGGSLRLSCAASGFTFSDYGMHWVRQAPGKGLEWVAFISS
GSSIIYYADIVKGRSTISRDNAKNTLYLQMNSLRAEDTAVYYCTRGNKRGFFDY
WGQGTLVTVSS
SEQ ID NO: 43.

In at least one embodiment, in the anti-IL-4R antibody or antigen-binding fragment:
the heavy chain variable region comprises the sequence set forth in SEQ ID NO: 1, and
the light chain variable region comprises the sequence set forth in SEQ ID NO: 2; or
the heavy chain variable region comprises the sequence set forth in SEQ ID NO: 9, and
the light chain variable region comprises the sequence set forth in SEQ ID NO: 10; or
the heavy chain variable region comprises the sequence set forth in SEQ ID NO: 43, and
the light chain variable region comprises the sequence set forth in SEQ ID NO: 37; or
the heavy chain variable region comprises the sequence set forth in SEQ ID NO: 43, and
the light chain variable region comprises the sequence set forth in SEQ ID NO: 41; or
the heavy chain variable region comprises the sequence set forth in SEQ ID NO: 47, and
the light chain variable region comprises the sequence set forth in SEQ ID NO: 48.

In some embodiments, the anti-IL-4R antibody or the antigen-binding fragment thereof comprises a heavy chain variable region and a light chain variable region, wherein:
the heavy chain variable region comprises:
   (I) the sequence set forth in any one of SEQ ID NOs: 25-27 or a sequence having at least 70%, 80%, 90%, 95%, 98% or 99% identity to any one of SEQ ID NOs: 25-27; or
   (II) the sequence set forth in any one of SEQ ID NOs: 31-33 or a sequence having at least 70%, 80%, 90%, 95%, 98% or 99% identity to any one of SEQ ID NOs: 31-33;
and/or, the light chain variable region comprises:
   (I) the sequence set forth in any one of SEQ ID NOs: 28-30 or a sequence having at least 70%, 80%, 90%, 95%, 98% or 99% identity to any one of SEQ ID NOs: 28-30; or
   (II) the sequence set forth in any one of SEQ ID NOs: 34-36 or a sequence having at least 70%, 80%, 90%, 95%, 98% or 99% identity to any one of SEQ ID NOs: 34-36.
hu25G7-VH-a: 
EVQLVESGGGLVQPGGSLRLSCAASGFTFSDYGMHWVRQAPGKGLEWVAFISS
GSSIIYYADIVKGRFTISRDNAKNSLYLQMNSLRAEDTAVYYCARGNKRGFFDY
WGQGTLVTVSS
SEQ ID NO: 25;
hu25G7-VH-b: 
EVQLVESGGGLVQPGGSLRLSCAASGFTFSDYGMHWVRQAPGKGLEWVAFISS
GSSIIYYADIVKGRFTISRDNAKNSLYLQMNSLRAEDTAVYYCTRGNKRGFFDY
WGQGTLVTVSS
SEQ ID NO: 26;
hu25G7-VH-c: 
EVQLVESGGGLVQPGGSLRLSCAASGFTFSDYGMHWVRQAPGKGLEWVAFISS
GSSIIYYADIVKGRSTISRDNAKNSLYLQMNSLRAEDTAVYYCTRGNKRGFFDY
WGQGTLVTVSS
SEQ ID NO: 27;
hu25G7-VL-a: 
EIVLTQSPATLSLSPGERATLSCNASSSVSYMYWYQQKPGQAPRLLIYLTSNLAS
GIPARFSGSGSGTDFTLTISSLEPEDFAVYYCQQWRSNPPMLTFGGGTKVEIK
SEQ ID NO: 28;
hu25G7-VL-b: 
EIVLTQSPATLSLSPGERATLSCNASSSVSYMYWYQQKPGQAPRLLIYLTSNLAS
GIPARFSGSGSGTDYTLTISSLEPEDFAVYYCQQWRSNPPMLTFGGGTKVEIK
SEQ ID NO: 29;
hu25G7-VL-c: 
EIVLTQSPATLSLSPGERATLSCNASSSVSYMYWYQQKPGQAPRPWIYLTSNLAS
GIPARFSGSGSGTDYTLTISSLEPEDFAVYYCQQWRSNPPMLTFGGGTKVEIK
SEQ ID NO: 30;
hu7B 10-VH-a: 
EVQLVQSGAEVKKPGASVKVSCKASGYTFTSYWMHWVRQAPGQGLEWMGLI
HPNSDTTKFSENFKTRVTMTRDTSISTAYMELSRLRSDDTAVYYCARSKIITTIVA
RHWYFDVWGQGTTVTVSS
SEQ ID NO: 31;
hu7B10-VH-b: 
EVQLVQSGAEVKKPGASVKVSCKASGYTFTSYWMHWVRQAPGQGLEWMGLI
HPNSDTTKFSENFKTRVTMTIDTSISTAYMELSRLRSDDTAVYYCAKSKIITTIVA
RHWYFDVWGQGTTVTVSS
 SEQ ID NO: 32;
hu7B 10-VH-c: 
EVQLVQSGAEVKKPGASVKVSCKASGYTFTSYWMHWVRQAPGQGLEWMGLI
HPNSDTTKFSENFKTRVTLTIDKSISTAYMELSRLRSDDTAVYYCAKSKIITTIVAR
HWYFDVWGQGTTVTVSS
SEQ ID NO: 33;
hu7B 10-VL-a: 
DIVMTQTPLSLSVTPGQPASISCKASQSVDYGGDSYMNWYLQKPGQPPQLLIYA
ASNLESGVPDRFSGSGSGTDFTLKISRVEAEDVGVYYCQHSNENPPTFGGGTKV
EIK
SEQ ID NO: 34;
hu7B 10-VL-b: 
DIVLTQTPLSLSVTPGQPASISCKASQSVDYGGDSYMNWYLQKPGQPPQLLIYAA
SNLESGVPDRFSGSGSGTDFTLKISRVEAEDVGVYYCQHSNENPPTFGGGTKVEI
K
SEQ ID NO: 35;
hu7B 10-VL-c:

DIVMTQTPLSLSVTPGQPASISCKASQSVDYGGDSYMNWYLQKPGQPPQLLIYA
ASNLESGIPDRFSGSGSGTDFTLKISRVEAEDVGVYYCQHSNENPPTFGGGTKVE
IK
SEQ ID NO: 36.

In some specific embodiments, the heavy chain variable region comprises the sequence set forth in any one of SEQ ID NOs: 25-27, and the light chain variable region comprises the sequence set forth in any one of SEQ ID NOs: 28-30; or
the heavy chain variable region comprises the sequence set forth in any one of SEQ ID NOs: 31-33, and the light chain variable region comprises the sequence set forth in any one of SEQ ID NOs: 34-36.

In some embodiments, the amino acid at position 44 of the heavy chain variable region (VH-44) and the amino acid at position 100 of the light chain variable region (VL-100) are optionally mutated into cysteine.

In some embodiments, the anti-IL-4R antibody or the antigen-binding fragment thereof comprises a heavy chain and a light chain, wherein the heavy chain comprises:
(I) the sequence set forth in SEQ ID NO: 17 or a sequence having at least 70%, 80%, 90%, 95%, 98% or 99% identity to SEQ ID NO: 17; or
(II) the sequence set forth in SEQ ID NO: 19 or a sequence having at least 70%, 80%, 90%, 95%, 98% or 99% identity to SEQ ID NO: 19; or
(III) the sequence set forth in SEQ ID NO: 44 or a sequence having at least 70%, 80%, 90%, 95%, 98% or 99% identity to SEQ ID NO: 44;
and/or the light chain comprises:
(I) the sequence set forth in SEQ ID NO: 18 or a sequence having at least 70%, 80%, 90%, 95%, 98% or 99% identity to SEQ ID NO: 18; or
(II) the sequence set forth in SEQ ID NO: 20 or a sequence having at least 70%, 80%, 90%, 95%, 98% or 99% identity to SEQ ID NO: 20; or
(III) the sequence set forth in SEQ ID NO: 45 or a sequence having at least 90%, 95%, 98% or 99% identity to SEQ ID NO: 45; or
(IV) the sequence set forth in SEQ ID NO: 46 or a sequence having at least 90%, 95%, 98% or 99% identity to SEQ ID NO: 46.

hu25G7 HC 
EVQLVESGGGLVQPGGSLRLSCAASGFTFSDYGMHWVRQAPGKGLEWVAFISS
GSSIIYYADIVKGRSTISRDNAKNSLYLQMNSLRAEDTAVYYCTRGNKRGFFDY
WGQGTLVTVSSASTKGPSVFPLAPCSRSTSESTAALGCLVKDYFPEPVTVSWNS
GALTSGVHTFPAVLQSSGLYSLSSVVTVPSSSLGTKTYTCNVDHKPSNTKVDKR
VESKYGPPCPPCPAPEFLGGPSVFLFPPKPKDTLMISRTPEVTCVVVDVSQEDPE
VQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAV
EWESNGQPENNYKTTPPVLDSDGSFFLYSRLTVDKSRWQEGNVFSCSVMHEAL
HNHYTQ KSLSLSLGK
SEQ ID NO: 17;
hu25G7 LC 
EIVLTQSPATLSLSPGERATLSCNASSSVSYMYWYQQKPGQAPRLLIYLTSNLAS
GIPARFSGSGSGTDFTLTISSLEPEDFAVYYCQQWRSNPPMLTFGGGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
SEQ ID NO: 18;
hu7B10 HC 
EVQLVQSGAEVKKPGASVKVSCKASGYTFTSYWMHWVRQAPGQGLEWMGLI
HPNSDTTKFSENFKTRVTMTIDTSISTAYMELSRLRSDDTAVYYCAKSKIITTIVA
RHWYFDVWGQGTTVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSN
TKVDKKVEPKSCDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWL
NGKEYKCKVSNKALPAPIEKTISKAKGQPREPQVYTLPPSREEMTKNQVSLTCL
VKGFYPSDIAVEWESNGQPENNYKTTPPVLDSDGSFFLYSKLTVDKSRWQQGN
VFSCSVMHEALHNHYTQKSLSLSPGK
SEQ ID NO: 19;
hu7B10 LC 
DIVLTQTPLSLSVTPGQPASISCKASQSVDYGGDSYMNWYLQKPGQPPQLLIYAA
SNLESGVPDRFSGSGSGTDFTLKISRVEAEDVGVYYCQHSNENPPTFGGGTKVEI
KRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC
SEQ ID NO: 20;
hu25G7 HC 
EVQLVESGGGLVQPGGSLRLSCAASGFTFSDYGMHWVRQAPGKGLEWVAFISS
GSSIIYYADIVKGRSTISRDNAKNTLYLQMNSLRAEDTAVYYCTRGNKRGFFDY
WGQGTLVTVSSASTKGPSVFPLAPCSRSTSESTAALGCLVKDYFPEPVTVSWNS
GALTSGVHTFPAVLQSSGLYSLSSVVTVPSSSLGTKTYTCNVDHKPSNTKVDKR
VESKYGPPCPPCPAPEFLGGPSVFLFPPKPKDTLMISRTPEVTCVVVDVSQEDPE
VQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAV
EWESNGQPENNYKTTPPVLDSDGSFFLYSRLTVDKSRWQEGNVFSCSVMHEAL
HNHYTQKSLSLSLGK
SEQ ID NO: 44;
hu25G7-ALC 
EIVLTQSPATLSLSPGERATLSCRASSSVPYMYWYQQKPGQAPRLLIYLTSNLAS
GIPARFSGSGSGTDFTLTISSLEPEDFAVYYCQQWRAYPPMLTFGGGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

SEQ ID NO: 45;
hu25G7-B LC

EIVLTQSPATLSLSPGERATLSCRASPGVPPLAWYQQKPGQAPRLLIYLASSRPSGI
PARFSGSGSGTDFTLTISSLEPEDFAVYYCQQWRSNPPMLTFGGGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
SEQ ID NO: 46.

In at least one embodiment, the heavy chain comprises the sequence set forth in SEQ ID NO: 17, and the light chain comprises the sequence set forth in SEQ ID NO: 18; or
the heavy chain comprises the sequence set forth in SEQ ID NO: 19, and the light chain comprises the sequence set forth in SEQ ID NO: 20; or
the heavy chain comprises the sequence set forth in SEQ ID NO: 44, and the light chain comprises the sequence set forth in SEQ ID NO: 45; or
the heavy chain comprises the sequence set forth in SEQ ID NO: 44, and the light chain comprises the sequence set forth in SEQ ID NO: 46.

In some embodiments, the anti-IL-4R antibody or antigen-binding fragment is a murine antibody, a chimeric antibody, a fully human antibody, a humanized antibody or a fragment thereof. In some specific embodiments, the anti-IL-4R antibody or antigen-binding fragment is humanized.

In some embodiments, the anti-IL-4R antibody or the antigen-binding fragment thereof comprises a FR region sequence derived from a human germline light chain template IGKV3-11*01 (SEQ ID NO: 22, used for antibody 25G7) or a back-mutated sequence having at least 95% identity thereto. In some specific embodiments, the back mutation is selected from one or more of 46P, 47W and 71Y

In some embodiments, the anti-IL-4R antibody or the antigen-binding fragment thereof comprises a FR region sequence derived from a human germline heavy chain template IGHV3-48*01 (SEQ ID NO: 21, used for antibody 25G7) or a back-mutated sequence having at least 95% identity thereto. In some specific embodiments, the back mutation is selected from one or more of 94A, 67S and 93T.

In some embodiments, the anti-IL-4R antibody or the antigen-binding fragment thereof comprises a FR region sequence derived from a human germline light chain template IGKV2D-29*01 (SEQ ID NO: 24, used for antibody 7B10) or a back-mutated sequence having at least 95% identity thereto. In some specific embodiments, the back mutation is selected from the group consisting of 4L and/or 58I.

In some embodiments, the anti-IL-4R antibody or the antigen-binding fragment thereof comprises a FR region sequence derived from a human germline heavy chain template IGHV1-2*02 (SEQ ID NO: 23, used for antibody 7B10) or a back-mutated sequence having at least 95% identity thereto. In some specific embodiments, the back mutation is selected from one or more of 69L, 71I, 73K and 94K.
Human germline heavy chain template IGHV3-48*01: 
EVQLVESGGGLVQPGGSLRLSCAASGFTFSSYSMNWVRQAPGKGLEWVSYISSS
SSTIYYADSVKGRFTISRDNAKNSLYLQMNSLRAEDTAVYYCAR
SEQ ID NO: 21;
Human germline light chain template IGKV3-11^{∗}01: 
EIVLTQSPATLSLSPGERATLSCRASQSVSSYLAWYQQKPGQAPRLLIYDASNRAT
GIPARFSGSGSGTDFTLTISSLEPEDFAVYYCQQRSNWP
SEQ ID NO: 22;
Human germline heavy chain template IGHV1-2^{∗}02: 
QVQLVQSGAEVKKPGASVKVSCKASGYTFTGYYMHWVRQAPGQGLEWMGWI
NPNSGGTNYAQKFQGRVTMTRDTSISTAYMELSRLRSDDTAVYYCAR
SEQ ID NO: 23;
Human germline light chain template IGKV2D-29^{∗}01:

DIVMTQTPLSLSVTPGQPASISCKSSQSLLHSDGKTYLYWYLQKPGQPPQLLIYE
VSNRFSGVPDRFSGSGSGTDFTLKISRVEAEDVGVYYCMQSIQLP
SEQ ID NO: 24.

In some embodiments, the anti-IL-4R antibody or the antigen-binding fragment thereof comprises a heavy chain constant region selected from the group consisting of the heavy chain constant regions of human IgG1, IgG2, IgG3 and IgG4, or a variant thereof. In some specific embodiments, the heavy chain constant region of human IgG1 or a variant thereof is comprised. In some embodiments, the anti-IL-4R antibody or the antigen-binding fragment thereof comprises the constant region of a human κ or λ chain or a variant thereof.

In some embodiments, provided is the anti-IL-4R antibody or the antigen-binding fragment thereof, wherein the antibody is a humanized antibody; the heavy chain comprises the sequence set forth in SEQ ID NO: 17 or a sequence having at least 85% sequence identity thereto, and the light chain comprises the sequence set forth in SEQ ID NO: 18 or a sequence having at least 85% sequence identity thereto.

In some embodiments, provided is the anti-IL-4R antibody or the antigen-binding fragment thereof, wherein the antibody is a humanized antibody; the heavy chain comprises the sequence set forth in SEQ ID NO: 19 or a sequence having at least 85% sequence identity thereto, and the light chain comprises the sequence set forth in SEQ ID NO: 20 or a sequence having at least 85% sequence identity thereto.

In some embodiments, provided is the anti-IL-4R antibody or the antigen-binding fragment thereof, wherein the antibody is a humanized antibody; the heavy chain comprises the sequence set forth in SEQ ID NO: 44 or a sequence having at least 85% sequence identity thereto, and the light chain comprises the sequence set forth in SEQ ID NO: 45 or a sequence having at least 85% sequence identity thereto.

In some embodiments, provided is the anti-IL-4R antibody or the antigen-binding fragment thereof, wherein the antibody is a humanized antibody; the heavy chain comprises the sequence set forth in SEQ ID NO: 44 or a sequence having at least 85% sequence identity thereto, and the light chain comprises the sequence set forth in SEQ ID NO: 46 or a sequence having at least 85% sequence identity thereto.

In some embodiments, provided is an isolated anti-IL-4R antibody or an antigen-binding fragment thereof, which competes for binding to human IL-4R or an epitope thereof with any one of the anti-IL-4R antibodies or the antigen-binding fragments thereof described above.

In some embodiments, provided is a bispecific antibody or a multispecific antibody, which comprises the light chain variable region and/or the heavy chain variable region of any one of the anti-IL-4R antibodies or the antigen-binding fragments thereof described above.

In some other embodiments, provided is a single-chain antibody, which comprises the light chain variable region and/or the heavy chain variable region of any one of the anti-IL-4R antibodies or the antigen-binding fragments thereof described above.

In some embodiments, the humanized anti-IL-4R antibody or the antigen-binding fragment thereof further comprises the heavy chain constant region of human IgG1, IgG2, IgG3 or IgG4, or a variant thereof. In at least one embodiment, the heavy chain constant region of human IgG2 or IgG4 is comprised because IgG2 or IgG4 has no ADCC toxicity. In another embodiment, IgG1 that has no ADCC (antibody-dependent cell-mediated cytotoxicity) toxicity after amino acid mutation is used. In at least one embodiment, the variant comprises a heavy chain constant region mutation leading to reduced or absent ADCC effector function, such as, but not limited to 297A, 234Aor 235A of IgG1.

In some embodiments, the anti-IL-4R antibody or the antigen-binding fragment thereof may be an antibody variant having 1 to 10 (e.g., 1, 2, 3, 4, 5, 6, 7, 8, 9 or 10) amino acid alterations in the light chain and/or 1 to 10 (e.g., 1, 2, 3, 4, 5, 6, 7, 8, 9 or 10) amino acid alterations in the heavy chain.

In some embodiments, the variant described above has identical or similar biological function or effect to the parent anti-IL-4R antibody or the fragment thereof.

In some embodiments, the anti-IL-4R antibody or the antigen-binding fragment thereof may be known, such as those described, for example, in WO2010053751, WO2001092340, WO2008054606, WO2014031610 and WO2020038454, each of which is incorporated herein by reference.

In some embodiments, the anti-IL-4R antibody or the antigen-binding fragment thereof includes, but is not limited to, Dupixent, PRS-060, AK-120, 63 IgG1, CBP201, AMG-317 or an antigen-binding fragment thereof.

In some embodiments, the anti-IL-4R antibody or the antigen-binding fragment thereof is an anti-human IL-4R antibody or an antigen-binding fragment thereof.

In some embodiments, the antigen-binding fragment includes, but is not limited to, Fab, Fab', Fv, F(ab')2, linear antibody, scFv (single-chain Fv antibody), tandem di-scFv, tandem tri-scFv, diabody, triabody, tetrabody, sdAb (single-domain antibody or nanobody), sdFv, peptibody, domain antibody, multispecific antibody (e.g., bispecific antibody, trispecific antibody or tetraspecific antibody), dsFv (disulfide-stabilized Fv) and ScdsFv (disulfide-stabilized single-chain Fv antibody).

In some embodiments, mutations for structural stabilization are introduced into the anti-IL-4R antibody or antigen-binding fragment; for example, some amino acid residues are mutated into cysteine residues. In some specific embodiments, the mutations occur in the framework region; in some specific embodiments, the mutations occur in the heavy chain variable region and/or the light chain variable region; in some specific embodiments, the mutations occur in the heavy or light chain variable region and the framework region. In some embodiments, the amino acid residues at positions selected from one or more of the following are mutated into cysteine residues: VL100 and VH44; VL101 and VH44; VL34 and VH100; VL43 and VH91; VL43 and VH103; VL49 and H100; VL87 and VH45; VL91 and VH98; VL91 and VH99; VL96 and VH47; and VL98 and VH45.

In some specific embodiments, the amino acid residues at positions selected from one or more of the following are mutated into cysteine residues: VL100 and VH44; VL43 and VH91; VL43 and VH103; and VL98 and VH45.

In one specific embodiment, the amino acids at positions VH44 and VL100 are mutated into cysteine.

Unless otherwise indicated, the amino acid sequence in the anti-IL-4R antibody or the antigen-binding protein thereof disclosed herein is numbered according to the Kabat numbering scheme.

In some embodiments, the anti-IL-4R antibody or antigen-binding fragment is a scFv or ScdsFv, with the heavy chain variable region (VH) and the light chain variable region (VL) linked by a linking peptide L2.

In some embodiments, the linking peptide L2 is greater than 12 amino acid residues in length and is rich in small, non-polar amino acid residues, polar amino acid residues and/or hydrophilic amino acid residues, e.g., glycine, serine and threonine.

In some embodiments, the linking peptide L2 may be selected from the group consisting of a variety of linkers known in the art, including "GS" linkers, e.g., (GxS)n, (SxG)n, (GGGGS)n or (G)n, where x is an integer from 1-6, and n is an integer from 1-30.

In some embodiments, non-limiting examples of the linking peptide L2 include GKSSGSGSESKS (SEQ ID NO: 54), EGKSSGSGSESKEF (SEQ ID NO: 55), GSTSGSGKSSEGKG (SEQ ID NO: 56), GSTSGSGKSSEGSGSTKG (SEQ ID NO: 57), GSTSGSGKPGSGEGSTKG (SEQ ID NO: 58), SRSSG (SEQ ID NO: 59) and SGSSC (SEQ ID NO: 60).

In some embodiments, the linking peptide L2 consists of a repeated GGGGS amino acid sequence (SEQ ID NO: 61) or a variant thereof, e.g., (GGGGS)n, where n may be 0, 1, 2, 3, 4, 5 or more; for example, n is 2, 3 or 4; for example, n is 3.

In some embodiments, the ScFv is constructed in the format of VH-L2-VL, which means that L2 is linked to the C-terminus of VH and to the N-terminus of VL;
in some embodiments, the ScFv is constructed in the format of VL-L2-VH, which means that L2 is linked to the C-terminus of VL and to the N-terminus of VH;
In some embodiments, the ScdsFv is constructed in the format of VH-L2-VL, which means that L2 is linked to the C-terminus of VH and to the N-terminus of VL;
In some embodiments, the ScdsFv is constructed in the format of VL-L2-VH, which means that L2 is linked to the C-terminus of VL and to the N-terminus of VH.

In some embodiments, the toxin is selected from the group consisting of: a bacterial toxin, an animal toxin and a plant toxin.

In some embodiments, the toxin includes a truncated toxin or a toxin variant, the toxin variant comprising an amino acid deletion, insertion, substitution or a combination thereof.

In some embodiments, the toxin includes a pore-forming toxin.

In some embodiments, the pore-forming toxin includes aerolysin from Aeromonas hydrophila or proaerolysin.

In some embodiments, the toxin includes bouganin, ricin, pseudomonas exotoxin (PE), cholera toxin or diphtheria toxin.

In some embodiments, the toxin includes, but is not limited to, a cytotoxic fragment or variant of pseudomonas exotoxin, for example, any one or more of PE-LR, PE-LO10R456A, PE-T20, PE-T20-KDEL, PE4E, PE40, PE38, PE24, PE25, PE38QQR, PE38KDEL and PE35 described in U.S. Patent Nos. 4,892,827; 5,512,658; 5,602,095; 5,608,039; 5,821,238; 5,854,044; 8,871,906; 8,907,060; 8,936,792; 9,346,859; 9,206,240; and 9,388,222, each of which is incorporated herein by reference.

In some embodiments, the toxin includes, for example, PE38KDEL set forth in SEQ ID NO: 49; variants with similar functions, such as PE38DKEL, PE38RDEL and PE38KNEL, may also be used.

The complex of the anti-IL-4R antibody or the antigen-binding fragment thereof disclosed herein optionally further comprises a linker L1.

The linker L1 links the toxin and the anti-IL-4R antibody or the antigen-binding fragment thereof.

Suitable linkers L1 generally allow each component of the present disclosure to fold as a three-dimensional structure that is very similar to the structure that the component form in the absence of any linkers or other components.

In some embodiments, the linker L1 is stable extracellularly, such that the ADC remains intact when present in an extracellular environment but can be cleaved when internalized in a cell such as a cancer cell.

In some embodiments, suitable linkers L1 may include, for example, protease-sensitive, environmental redox potential-sensitive, pH-sensitive, acid-cleavable, photo-cleavable and/or heat-sensitive linkers.

In some embodiments, the linker L1 may be non-proteinaceous, e.g., a chemical linker. Examples of non-proteinaceous chemical linkers include, but are not limited to: N-succinimidyl(4-iodoacetyl)-aminobenzoate, S-(N-succinimidyl)thioacetate (SATA), N-succinimidyl-oxycarbonyl-cu-methyl-a-(2-pyridyldithio)toluene (SMPT), N-succinimidyl 4-(2-pyridyldithio)-pentanoate (SPP), succinimidyl 4-(N-maleimidomethyl)cyclohexanecarboxylate (SMCC or MCC), sulfosuccinimidyl(4-iodoacetyl)-aminobenzoate, 4-succinimidyl-oxycarbonyl-α-(2-pyridyldithio)toluene, sulfosuccinimidyl-6-(α-methyl-α-(pyridyldithiol)-toluamido)hexanoate, N-succinimidyl-3-(-2-pyridyldithio)-propionate (SPDP), succinimidyl 6(3(-(-2-pyridyldithio)-propanamido)hexanoate, sulfosuccinimidyl 6(3(-(-2-pyridyldithio)-propanamido)hexanoate, maleimidocaproyl (MC), maleimidocaproyl-valine-citrulline-p-aminobenzyloxycarbonyl (MC-vc-PAB), 3-maleimidobenzoic acid N-hydroxysuccinimide ester (MBS), α-alkyl derivatives, sulfo NHS-ATMBA (sulfosuccinimidyl N-[3-(acetylthio)-3-methylbutyryl-β-alanine]), sulfodichlorophenol, 2-iminothiolane, 3-(2-pyridyldithio)-propionylhydrazide, Ellman's reagent, dichlorotriazinic acid and S-(2-thiopyridyl)-L-cysteine.

In some embodiments, the complex formed with the anti-IL-4R antibody or the antigen-binding fragment thereof and the toxin is in the form of a fusion protein.

In some embodiments, the linker L1 is a proteinaceous linker, including but not limited to 1 or more amino acids or polypeptides. The linker L1 typically comprises about 2-50 amino acid residues, for example about 5-30 amino acid residues. Typically, the proteinaceous linker comprises the majority of amino acid residues of polar, uncharged and/or charged residues, e.g., threonine, proline, glutamine, glycine and alanine. Non-limiting examples of proteinaceous linkers include alanine-serine-glycine-proline-glutamic acid (ASGGPE) (SEQ ID NO: 50), valine-methionine (VM), alanine-methionine (AM), and AM(G2-4S)xAM, where G is glycine, S is serine, and x is an integer from 1-10.

In some embodiments, the linker L1 is selected from the group consisting of the following peptides listed in standard one-letter codes: ASGCGPE (SEQ ID NO: 62), ASGCCGPE (SEQ ID NO: 63), ASGCGSCPE (SEQ ID NO: 64), ASCGTTGCPE (SEQ ID NO: 65), KASGKKYGCKKGPE (SEQ ID NO: 66) and KGGGCAGGPE (SEQ ID NO: 67).

Each of the components of the complex disclosed herein, e.g., the anti-IL-4R antibody or antigen-binding fragment, the linker L1 and the toxin, may be suitably linked or fused to each other in a way well known in the art and/or described herein.

In some embodiments, the anti-IL-4R antibody or antigen-binding fragment is linked or fused to the toxin by the linker L1.

In some embodiments, the C-terminus of the anti-IL-4R antibody or antigen-binding fragment is linked or fused to the linker L 1. In some embodiments, the N-terminus of the anti-IL-4R antibody or antigen-binding fragment is linked or fused to the linker L1. In some embodiments, the C-terminus and N-terminus of the anti-IL-4R antibody or antigen-binding fragment are linked or fused to identical or different linker L1, respectively.

In some embodiments, the toxin is pseudomonas exotoxin or a truncated fragment or toxin variant of pseudomonas exotoxin.

In some embodiments, the toxin is PE38, a truncated fragment of pseudomonas exotoxin.

In some embodiments, the toxin is PE38KDEL set forth in SEQ ID NO: 49; variants with similar functions, such as PE38DKEL, PE38RDEL and PE38KNEL, may also be used.

In some embodiments, L1 is a proteinaceous linker.

In some embodiments, L1 is selected from the group consisting of ASGGPE (SEQ ID NO: 50), ASGCCGPE (SEQ ID NO: 63), ASGCGSCPE (SEQ ID NO: 64), ASCGTTGCPE (SEQ ID NO: 65), KASGKKYGCKKGPE (SEQ ID NO: 66), KGGGCAGGPE (SEQ ID NO: 67), and AM (G₂₋₄S)ₓAM, where x is an integer from 1-10.

In some embodiments, the ScFv specifically binds to IL-4R, wherein VH and VL are linked by a linking peptide L2, and VH and VL comprise mutations for stabilizing the structure of the antibody which are introduced into the framework region. In some embodiments, the amino acids at positions VH-44 and VL-100 are mutated into cysteine. In some embodiments, the ScFv is constructed in the format of VH-L2-VL or VL-L2-VH.

L2 consists of a repeated GGGGS amino acid sequence or a variant thereof, e.g., (GGGGS)n, where n may be 0, 1, 2, 3, 4, 5 or more; for example, n is 2, 3 or 4; for example, n is 3.

In some embodiments, the VH in the ScFv described above comprises:
(I) a HCDR1, a HCDR2 and a HCDR3 whose amino acid sequences are set forth in SEQ ID NO: 3, SEQ ID NO: 4 and SEQ ID NO: 5, respectively; or
(II) a HCDR1, a HCDR2 and a HCDR3 whose amino acid sequences are set forth in SEQ ID NO: 11, SEQ ID NO: 12 and SEQ ID NO: 13, respectively;
and/or the VL comprises:
(I) a LCDR1, a LCDR2 and a LCDR3 whose amino acid sequences are set forth in SEQ ID NO: 6, SEQ ID NO: 7 and SEQ ID NO: 8, respectively; or
(II) a LCDR1, a LCDR2 and a LCDR3 whose amino acid sequences are set forth in SEQ ID NO: 14, SEQ ID NO: 15 and SEQ ID NO: 16, respectively; or
(III) a LCDR1, a LCDR2 and a LCDR3 set forth in SEQ ID NO: 38, SEQ ID NO: 7 and SEQ ID NO: 40, respectively; or
(IV) a LCDR1, a LCDR2 and a LCDR3 set forth in SEQ ID NO: 42, SEQ ID NO: 39 and SEQ ID NO: 8, respectively.

In some embodiments, the ScFv described above comprises any one selected from the group consisting of (I) to (IV):
(I) a VH comprising a HCDR1, a HCDR2 and a HCDR3 whose amino acid sequences are set forth in SEQ ID NO: 3, SEQ ID NO: 4 and SEQ ID NO: 5, respectively; and
   a VL comprising a LCDR1, a LCDR2 and a LCDR3 whose amino acid sequences are set forth in SEQ ID NO: 6, SEQ ID NO: 7 and SEQ ID NO: 8, respectively;
(II) a VH comprising a HCDR1, a HCDR2 and a HCDR3 whose amino acid sequences are set forth in SEQ ID NO: 11, SEQ ID NO: 12 and SEQ ID NO: 13, respectively; and a VL comprising a LCDR1, a LCDR2 and a LCDR3 whose amino acid sequences are set forth in SEQ ID NO: 14, SEQ ID NO: 15 and SEQ ID NO: 16, respectively;
(III) a VH comprising a HCDR1, a HCDR2 and a HCDR3 whose amino acid sequences are set forth in SEQ ID NO: 3, SEQ ID NO: 4 and SEQ ID NO: 5, respectively; and
   a VL comprising a LCDR1, a LCDR2 and a LCDR3 whose amino acid sequences are set forth in SEQ ID NO: 38, SEQ ID NO: 7 and SEQ ID NO: 40, respectively;
(IV) a VH comprising a HCDR1, a HCDR2 and a HCDR3 whose amino acid sequences are set forth in SEQ ID NO: 3, SEQ ID NO: 4 and SEQ ID NO: 5, respectively; and
   a VL comprising a LCDR1, a LCDR2 and a LCDR3 whose amino acid sequences are set forth in SEQ ID NO: 42, SEQ ID NO: 39 and SEQ ID NO: 8, respectively.

In some embodiments, in the ScFv described above:
the VH comprises:
(I) the sequence set forth in SEQ ID NO: 1 or a sequence having at least 70%, 80%, 90%, 95%, 98% or 99% identity to SEQ ID NO: 1; or
(II) the sequence set forth in SEQ ID NO: 9 or a sequence having at least 70%, 80%, 90%, 95%, 98% or 99% identity to SEQ ID NO: 9; or
(III) the sequence set forth in SEQ ID NO: 43 or a sequence having at least 70%, 80%, 90%, 95%, 98% or 99% identity to SEQ ID NO: 43; or
and/or, the VL comprises:
(I) the sequence set forth in SEQ ID NO: 2 or a sequence having at least 70%, 80%, 90%, 95%, 98% or 99% identity to SEQ ID NO: 2; or
(II) the sequence set forth in SEQ ID NO: 10 or a sequence having at least 70%, 80%, 90%, 95%, 98% or 99% identity to SEQ ID NO: 10; or
(III) the sequence set forth in SEQ ID NO: 37 or a sequence having at least 70%, 80%, 90%, 95%, 98% or 99% identity to SEQ ID NO: 37; or
(IV) the sequence set forth in SEQ ID NO: 41 or a sequence having at least 70%, 80%, 90%, 95%, 98% or 99% identity to SEQ ID NO: 41.

Optionally, the VH and VL comprise mutations for stabilizing the structure of the antibody which are introduced into the framework region; in some embodiments, the amino acids at positions VH-44 and VL-100 are mutated into cysteine.

In at least one embodiment, in the ScFv, the VH comprises the sequence set forth in SEQ ID NO: 1, and the VL comprises the sequence set forth in SEQ ID NO: 2; or
the VH comprises the sequence set forth in SEQ ID NO: 9, and the VL comprises the sequence set forth in SEQ ID NO: 10; or
the VH comprises the sequence set forth in SEQ ID NO: 43, and the VL comprises the sequence set forth in SEQ ID NO: 37; or
the VH comprises the sequence set forth in SEQ ID NO: 43, and the VL comprises the sequence set forth in SEQ ID NO: 41;
optionally, the VH and VL comprise mutations for stabilizing the structure of the antibody which are introduced into the framework region.

In some embodiments, the amino acids at positions VH-44 and VL-100 are mutated into cysteine. The VH comprises the sequence set forth in SEQ ID NO: 47, and the VL comprises the sequence set forth in SEQ ID NO: 48.

In some embodiments, in the ScFv:
the VH comprises:
(I) the sequence set forth in any one of SEQ ID NOs: 25-27 or a sequence having at least 70%, 80%, 90%, 95%, 98% or 99% identity to any one of SEQ ID NOs: 25-27; or
(II) the sequence set forth in any one of SEQ ID NOs: 31-33 or a sequence having at least 70%, 80%, 90%, 95%, 98% or 99% identity to any one of SEQ ID NOs: 31-33;
and/or the VL comprises:
(I) the sequence set forth in any one of SEQ ID NOs: 28-30 or a sequence having at least 70%, 80%, 90%, 95%, 98% or 99% identity to any one of SEQ ID NOs: 28-30; or
(II) the sequence set forth in any one of SEQ ID NOs: 34-36 or a sequence having at least 70%, 80%, 90%, 95%, 98% or 99% identity to any one of SEQ ID NOs: 34-36;
optionally, the VH and VL comprise mutations for stabilizing the structure of the antibody which are introduced into the framework region. In some embodiments, the amino acids at positions VH-44 and VL-100 are mutated into cysteine.

In some specific embodiments, the VH comprises the sequence set forth in any one of SEQ ID NOs: 25-27, and VL comprises the sequence set forth in any one of SEQ ID NOs: 28-30; the amino acids at positions VH-44 and VL-100 are mutated into cysteine; or
the VH comprises the sequence set forth in any one of SEQ ID NOs: 31-33, and VL comprises the sequence set forth in any one of SEQ ID NOs: 34-36; the amino acids at positions VH-44 and VL-100 are mutated into cysteine.

Optionally, the VH and VL comprise mutations for stabilizing the structure of the antibody which are introduced into the framework region. In some embodiments, the amino acids at positions VH-44 and VL-100 are mutated into cysteine.

In some embodiments, the ScFv comprises a heavy chain and a light chain, wherein the heavy chain comprises:
(I) the sequence set forth in SEQ ID NO: 17 or a sequence having at least 70%, 80%, 90%, 95%, 98% or 99% identity to SEQ ID NO: 17; or
(II) the sequence set forth in SEQ ID NO: 19 or a sequence having at least 70%, 80%, 90%, 95%, 98% or 99% identity to SEQ ID NO: 19; or
(III) the sequence set forth in SEQ ID NO: 44 or a sequence having at least 70%, 80%, 90%, 95%, 98% or 99% identity to SEQ ID NO: 44;
and/or the light chain comprises:
(I) the sequence set forth in SEQ ID NO: 18 or a sequence having at least 70%, 80%, 90%, 95%, 98% or 99% identity to SEQ ID NO: 18; or
(II) the sequence set forth in SEQ ID NO: 20 or a sequence having at least 70%, 80%, 90%, 95%, 98% or 99% identity to SEQ ID NO: 20; or
(III) the sequence set forth in SEQ ID NO: 45 or a sequence having at least 90%, 95%, 98% or 99% identity to SEQ ID NO: 45; or
(IV) the sequence set forth in SEQ ID NO: 46 or a sequence having at least 90%, 95%, 98% or 99% identity to SEQ ID NO: 46;
optionally, the VH and VL comprise mutations for stabilizing the structure of the antibody which are introduced into the framework region. In some embodiments, the amino acids at positions VH-44 and VL-100 are mutated into cysteine.

In at least one embodiment, the heavy chain variable region VH comprises the sequence set forth in SEQ ID NO: 17, and the light chain variable region VL comprises the sequence set forth in SEQ ID NO: 18; or
the VH comprises the sequence set forth in SEQ ID NO: 19, and VL comprises the sequence set forth in SEQ ID NO: 20, wherein the amino acids at positions VH-44 and VL-100 are mutated into cysteine; or
the VH comprises the sequence set forth in SEQ ID NO: 44, and VL comprises the sequence set forth in SEQ ID NO: 45, wherein the amino acids at positions VH-44 and VL-100 are mutated into cysteine; or
the VH comprises the sequence set forth in SEQ ID NO: 44, and VL comprises the sequence set forth in SEQ ID NO: 46, wherein the amino acids at positions VH-44 and VL-100 are mutated into cysteine.

In some embodiments, the complex of the anti-IL-4R antibody or the antigen-binding fragment thereof has the sequence set forth in SEQ ID NO: 52 or SEQ ID NO: 53.

The present disclosure also provides a complex of an anti-IL-4R antibody or an antigen-binding fragment thereof, which comprises: the anti-IL-4R antibody or the antigen-binding fragment thereof and one or more toxins; wherein the toxins are selected from the group consisting of a pore-forming toxin, aerolysin from Aeromonas hydrophila, proaerolysin, bouganin, ricin, pseudomonas exotoxin, cholera toxin and diphtheria toxin; preferably, the toxins are selected from the group consisting of PE-LR, PE-LO10R456A, PE-T20, PE-T20-KDEL, PE4E, PE40, PE38, PE24, PE25, PE38QQR, PE35, PE38KDEL, PE38DKEL, PE38RDEL and PE38KNEL; more preferably, the toxins are PE38KDEL set forth in SEQ ID NO: 49.

The present disclosure provides a polynucleotide encoding any one of the complexes of the anti-IL-4R antibody or the antigen-binding fragment thereof described in the present disclosure. In some embodiments, the polynucleotide is DNA or RNA.

The present disclosure provides a vector comprising the polynucleotide described above, which is a eukaryotic expression vector, a prokaryotic expression vector or a viral vector. The present disclosure provides a host cell comprising the vector described above, and the host cell is selected from the group consisting of a prokaryotic cell and a eukaryotic cell. In at least one embodiment, the prokaryotic cell is selected from a bacterium, such as *E. coli.* In at least one embodiment, the eukaryotic cell is selected from the group consisting of a yeast and a mammalian cell, such as *Pichia pastoris* or a CHO cell or a human embryonic kidney (HEK) 293 cell.

The present disclosure provides a method for preparing a complex of an anti-IL-4R antibody or an antigen-binding fragment thereof, which comprises the following steps: expressing the complex of the anti-IL-4R antibody or the antigen-binding fragment thereof in the host cell described above, and isolating the complex of the anti-IL-4R antibody or the antigen-binding fragment thereof from the host cell, and optionally purifying the complex of the anti-IL-4R antibody or the antigen-binding fragment thereof.

### Pharmaceutical Composition

The present disclosure further provides a pharmaceutical composition comprising the complex of the anti-IL-4R antibody or the antigen-binding fragment thereof described above as an active ingredient, which is used as a medicament.

The pharmaceutical composition of the present disclosure may comprise, in addition to the active ingredient, one or more pharmaceutically acceptable excipients, diluents or vehicles. The pharmaceutical composition may comprise 0.1 to 99 wt.% of the active ingredient.

The present disclosure further provides use of any one or a combination selected from the group consisting of the following in the preparation of a medicament: the anti-IL-4R antibody or the antigen-binding fragment thereof according to the present disclosure, the complex of the anti-IL-4R antibody or the antigen-binding fragment thereof, and the pharmaceutical composition according to the present disclosure, wherein the antibody or the antigen-binding fragment thereof or the complex thereof is for use in treating and/or preventing a proliferative disease or delaying the progression of a proliferative disease. The proliferative disorder may be a cancer or tumor; for example, the cancer or tumor is a cancer or tumor associated with IL-4R expression. The cancer or tumor is selected from the group consisting of prostate cancer, ovarian cancer, breast cancer, endometrial cancer, multiple myeloma, melanoma, lymphoma (e.g., Hodgkin lymphoma, non-Hodgkin lymphoma or recurrent anaplastic large cell lymphoma), lung cancer, kidney cancer, liver cancer (e.g., small cell lung cancer and non-small cell lung cancer), large intestine cancer (e.g., colon cancer), pancreatic cancer, stomach cancer, leukemia (e.g., acute lymphocytic leukemia, acute myelocytic leukemia, acute promyelocytic leukemia, chronic myelocytic leukemia or chronic lymphocytic leukemia), brain cancer and a central nervous system tumor.

In some embodiments, the central nervous system tumor includes glioma, glioblastoma, astrocytoma, medulloblastoma, craniopharyngioma, ependymoma, pinealoma, hemangioblastoma, acoustic neuroma, oligodendroglioma, hemangioma, meningioma, neuroblastoma, retinoblastoma and adult blastoma.

In some embodiments, the central nervous system tumor is a glioblastoma.

In some embodiments, the glioblastoma is a recurrent or refractory glioblastoma.

In some embodiments, in the glioblastoma, O6-methylguanine-DNA methyhransferase (MGMT) expression is positive or negative.

### Treatment Method

The present disclosure provides a method for treating and/or preventing a proliferative disease or delaying progression of the proliferative disease, which comprises administering to a subject in need thereof the anti-IL-4R antibody or the antigen-binding fragment thereof according to the present disclosure, or the pharmaceutical composition according to the present disclosure, or the complex of the anti-IL-4R antibody or the antigen-binding fragment thereof according to the present disclosure in an amount effective in treating or delaying the disease, wherein the proliferative disorder may be a cancer or tumor.

The present disclosure provides a method for enhancing immune function in a subject having, suspected of having, or susceptible to a cell proliferative disorder. In some embodiments, the cell proliferative disorder is a cancer or tumor.

For example, the cancer or tumor is a cancer or tumor associated with IL-4R expression. The cancer or tumor is selected from the group consisting of prostate cancer, ovarian cancer, breast cancer, endometrial cancer, multiple myeloma, melanoma, lymphoma (e.g., Hodgkin lymphoma, non-Hodgkin lymphoma or recurrent anaplastic large cell lymphoma), lung cancer, kidney cancer, liver cancer (e.g., small cell lung cancer and non-small cell lung cancer), large intestine cancer (e.g., colon cancer), pancreatic cancer, stomach cancer, leukemia (e.g., acute lymphocytic leukemia, acute myelocytic leukemia, acute promyelocytic leukemia, chronic myelocytic leukemia or chronic lymphocytic leukemia), brain cancer and a central nervous system tumor.

In some embodiments, the central nervous system tumor includes glioma, glioblastoma, astrocytoma, medulloblastoma, craniopharyngioma, ependymoma, pinealoma, hemangioblastoma, acoustic neuroma, oligodendroglioma, hemangioma, meningioma, neuroblastoma, retinoblastoma and adult blastoma.

In some embodiments, the central nervous system tumor is a glioblastoma.

In some embodiments, the glioblastoma is a recurrent or refractory glioblastoma.

In some embodiments, in the glioblastoma, O6-methylguanine-DNA methyhransferase (MGMT) expression is positive or negative.

The dose of the compound (e.g., the complex or composition of the present disclosure) used in the therapeutic and/or prophylactic method of the present disclosure generally varies depending on the severity of the disease, the body weight of the subject and the relative efficacy of the compound. As a general guide, a suitable unit dose may be 0.1 mg to 1000 mg.

As is well known to those skilled in the art, the dosage of the drug administered depends on a variety of factors, including but not limited to, the activity of the particular compound employed, the age of the subject, the body weight of the subject, the health condition of the subject, the behavior of the subject, the diet of the subject, the time of administration, the route of administration, the rate of excretion, the combination of drugs, and the like. The dosage can be verified according to well-known treatment regimens.

### BRIEF DESCRIPTION OF THE DRAWINGS

FIG. 1A and FIG. 1B. Schematic diagrams of the structures of exemplary complexes: 25G7-scdsFv-PE38KDEL (FIG. 1A), which is an antibody conjugate drug of the scdsFv of the antibody 25G7 and the toxin PE38KDEL, and 25G7-scFv-PE38KDEL (FIG. 1B).
FIG. 2A and FIG. 2B. SDS-PAGE patterns of purified complexes 25G7-scdsFv-PE38KDEL and 25G7-scFv-PE38KDEL, where M is a molecular weight marker.
FIG. 3. Flow cytometry assays for IL-4R expression levels in glioblastoma (GBM) cell lines. LN229, U251 and U87 cerebral glioma cell strains stably transfected with human IL-4R are constructed using a lentivirus system, and the overexpression levels of IL-4R are determined by flow cytometry, with corresponding cells which are not transfected with human IL-4R constructs as controls.
FIG. 4A to FIG. 4C. Flow cytometry assays of complexes for binding on the surface of glioblastoma cells. 25G7-scdsFv-PE38KDEL and MDNA55 are assayed by flow cytometry for binding ability on the cell surface of LN229-IL4R, U251-IL4R and U87-IL4R cell strains stably transfected with IL-4R.
FIG. 5A to FIG. 5C. The endocytosis efficiency of complexes in glioblastoma cells; 25G7-scdsFv-PE38KDEL (25G7-IT) and MDNA55 are assayed by flow cytometry for 0-to-24-hour endocytosis efficiency in LN229-IL4R, U251-IL4R and U87-IL4R cells are determined.
FIG. 6A to FIG. 6F. The *in vitro* cytotoxicity of complexes to glioblastoma cells; 25G7-scdsFv-PE38KDEL (25G7-IT) and MDNA55 are assayed by the CTG method for *in vitro* cytotoxicity to LN229-IL4R, U251-IL4R, U87-IL4R and control cells.
FIG. 7A to FIG. 7D. The CTG method is used to compare the *in vitro* cytotoxicity results of 25G7-scFv-PE38KDEL, 25G7-scdsFv-PE38KDEL and MDNA55 in LN229-IL4R, U251-IL4R and control glioma cells.
FIG. 8A to FIG. 8C. Comparison of the *in vivo* efficacy of complexes in a nude mouse tumor model subcutaneously inoculated with U87-MG; 25G7-scdsFv-PE38KDEL (25G7-IT) and MDNA55 are both administered at a dose of 0.5 mg/kg (mpk), intratumorally (i.t.).
FIG. 9A to FIG. 9C. Comparison of the *in vivo* efficacy of complexes in a nude mouse tumor model subcutaneously inoculated with LN229-IL4R (IL-4R-overexpressing LN229 cells); 25G7-scdsFv-PE38KDEL (25G7-IT) and MDNA55 are both administered at a dose of 0.25 mpk (i.t.), or 25G7-scdsFv-PE38KDEL at 0.25 mpk (i.v.).
FIG. 10A to FIG. 10C. Comparison of the *in vivo* efficacy of complexes in a nude mouse tumor model subcutaneously inoculated with LN229-IL4R; 25G7-scdsFv-PE38KDEL (25G7-IT) is administered at doses of 0.1 mpk (the second dose and the following doses are reduced to 0.05 mpk) and 0.3 mpk (the second dose and the following doses are reduced to 0.15 mpk), and MDNA55 is administered at a dose of 0.1 mpk (the second dose and the following doses are reduced to 0.05 mpk), intratumorally (i.t.).

### DETAILED DESCRIPTION

### Terms

To facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure belongs.

The present disclosure incorporates the disclosure of the patent document WO2020/038454A1 in its entirety into the present application.

The three-letter and single-letter codes for amino acids used in the present disclosure are described in J. Biol. Chem, 243, p3558 (1968).

"Human IL-4R" (hIL-4R) refers to a human cytokine receptor that specifically binds to interleukin-4 (IL-4) or IL-4Rα. hIL-4R is intended to encompass various forms of molecules of IL-4R in various stages *in vivo,* such as, but not limited to, molecules produced by the IL-4R gene during amplification, replication, transcription, splicing, processing, translation and modification, e.g., precursor IL-4R, mature IL-4R, naturally-occurring IL-4R splice variants, modified IL-4R, or fragments thereof.

The term "antibody" refers to an immunoglobulin, which is of a tetrapeptide chain structure formed by connecting two identical heavy chains and two identical light chains by interchain disulfide bonds. The heavy chain constant regions of an immunoglobulin differ in their amino acid composition and arrangement, and thus in their antigenicity. Accordingly, immunoglobulins can be divided into five classes, or isotypes of immunoglobulins, namely IgM, IgD, IgG, IgA and IgE, with their corresponding heavy chains being µ chain, δ chain, γ chain, α chain and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3 and IgG4. Light chains are divided into κ or λ chains according to differences in the constant regions. Each of the five classes of Ig may have a κ chain or λ chain.

The antibody light chain may further comprise a light chain constant region comprising human or murine κ and λ chains or variants thereof.

The antibody heavy chains may further comprise a heavy chain constant region comprising human or murine IgG1, IgG2, IgG3 and IgG4 or variants thereof.

In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (HVRs) and 4 framework regions (FRs) with relatively conservative sequences. The 3 hypervariable regions determine the specificity of the antibody and thus are also known as complementarity determining regions (CDRs). Each light chain variable region (LCVR) or heavy chain variable region (HCVR) consists of 3 CDRs and 4 FRs arranged from the amino-terminus to the carboxyl-terminus in the following order: FR1, CDR1, FR2, CDR2, FR3, CDR3 and FR4. The 3 CDR regions of the light chain refer to light chain complementarity determining region 1 (LCDR1), light chain complementarity determining region 2 (LCDR2), and light chain complementarity determining region 3 (LCDR3); the 3 CDR regions of the heavy chain refer to heavy chain complementarity determining region 1 (HCDR1), heavy chain complementarity determining region 2 (HCDR2) and heavy chain complementarity determining region 3 (HCDR3).

The antibody includes murine antibodies, chimeric antibodies, humanized antibodies and fully human antibodies, which may be recombinantly obtained, for example, may be recombinant fully human antibodies obtained by affinity maturation.

"Recombinant human antibodies" include fully human antibodies that are prepared, expressed, created or isolated by recombinant methods, and the techniques and methods involved are well known in the art; examples are (1) antibodies isolated from an animal (e.g., a mouse) that is transgenic or transchromosomal for human immunoglobulin genes or a hybridoma prepared therefrom; (2) antibodies isolated from a host cell transformed to express the antibodies, e.g., from a transfectoma; (3) antibodies isolated from a recombinant, combinatorial human antibody library; and (4) antibodies prepared, expressed, created or isolated by methods such as splicing of human immunoglobulin gene sequences to other DNA sequences. Such recombinant fully human antibodies comprise variable and constant regions that utilize specific human germline immunoglobulin sequences encoded by germline genes, and also include subsequent rearrangements and mutations which occur, for example, during antibody maturation.

The term "host cell" refers to a cell into which an expression vector has been introduced. Host cells may include bacterial, microbial, plant or animal cells. Bacteria susceptible to transformation include members of the species *enterobacteria,* such as strains of *Escherichia coli* or *Salmonella*; *Bacillaceae* such as *Bacillus subtilis*; *Pneumococcus*; *Streptococcus* and *Haemophilus influenzae.* Suitable microorganisms include *Saccharomyces cerevisiae* and *Pichia pastoris.* Suitable animal host cell lines include CHO (Chinese hamster ovary cell line) and NS0 cells.

"Murine antibody" as used herein refers to a monoclonal antibody to human IL-4R prepared according to the knowledge and skill in the art. During the preparation, a test subject is injected with the IL-4R antigen (or a polypeptide comprising epitopes), and then hybridoma of antibodies expressing the desired sequence or functional properties is isolated. In some embodiments, the murine human IL-4R-binding antibody or the antigen-binding fragment thereof may further comprise a light chain constant region of a murine κ or λ chain or a variant thereof, or further comprise a heavy chain constant region of a murine IgG1, IgG2, IgG3 or IgG4 or a variant thereof.

"Fully human antibody" includes antibodies having variable and constant regions derived from human germline immunoglobulin sequences. The fully human antibody herein may include amino acid residues not encoded by human germline immunoglobulin sequences (e.g., mutations introduced by random or site-specific mutagenesis *in vitro* or by somatic mutations *in vivo*). However, the term "fully human antibody" does not include antibodies in which CDR sequences derived from the germline of another mammalian species (such as mice) have been grafted into a human framework sequence (i.e., "humanized antibodies").

"Humanized antibody", also known as a CDR-grafted antibody, refers to an antibody produced by grafting non-human CDR sequences into the framework of variable regions of a human antibody. Such an antibody can overcome the strong immune response induced by the chimeric antibody because of carrying a large amount of heterologous protein components. To avoid the decrease in activity caused by the decrease in immunogenicity, the variable regions of a human antibody can be subjected to minimum reverse mutation to maintain activity.

The term "chimeric antibody" refers to an antibody obtained by fusing a variable region of a murine antibody and a constant region of a human antibody, which can reduce an immune response induced by the murine antibody. The chimeric antibody is established by firstly establishing hybridoma secreting murine specific monoclonal antibody, then cloning a variable region gene from the mouse hybridoma cells, cloning a constant region gene of human antibody as required, linking the mouse variable region gene and the human constant region gene into a chimeric gene, inserting the chimeric gene into a human vector, and finally expressing chimeric antibody molecules in a eukaryotic industrial system or prokaryotic industrial system. The constant region of the human antibody may be selected from the group consisting of the heavy chain constant regions of human IgG1, IgG2, IgG3 and IgG4 or variants thereof, for example comprising human IgG2 or IgG4 heavy chain constant regions, or IgG1 mutated at amino acids without ADCC (antibody-dependent cell-mediated cytotoxicity) toxicity.

"Antigen-binding fragment" refers to Fab fragments; Fab' fragments; F(ab')2 fragments; Fv fragments and scFv fragments that bind to human IL-4R; and polypeptides or proteins comprising the fragments; which have antigen-binding activity. The "antigen-binding fragment" comprises one or more of the CDR regions of the antibody described herein. The Fv fragment comprises the heavy chain variable region and the light chain variable region of the antibody but does not comprise the constant region, and has the smallest antibody fragment of the entire antigen-binding sites. Generally, the Fv antibody also comprises a polypeptide linker between the VH and VL domains, and is capable of forming the structure required for antigen binding. Two antibody variable regions can also be linked into a single polypeptide chain using different linkers, known as single chain antibody or single chain Fv (scFv).

The term "single chain antibody", "single chain Fv" or "scFv" refers to a molecule comprising a heavy chain variable domain (or region; VH) and a light chain variable domain (or region; VL) linked by a linker (or a linking peptide). Such scFv molecules may have a general structure: NH2-VL-linker-VH-COOH or NH2-VH-linker-VL-COOH. Suitable linkers in the prior art consist of repeated GGGGS amino acid sequences or variants thereof, for example, 1-4 repeated variants (Holliger et al. (1993), Proc. Natl. Acad. Sci. USA 90:6444-6448). Other linkers that can be used in the present disclosure are described in Alfthan et al. (1995), Protein Eng. 8:725-731; Choi et al. (2001), Eur. J. Immunol. 31:94-106; Hu et al. (1996), Cancer Res. 56:3055-3061; Kipriyanov et al. (1999), J. Mol. Biol. 293:41-56; and Roovers et al. (2001), Cancer Immunol.

The term "antibody framework" refers to a portion of a variable domain VL or VH, which serves as a framework for the antigen-binding loops (CDRs) of the variable domain. It is essentially a variable domain without CDRs.

"Bind to IL-4R" refers to the ability to interact with human IL-4R (or epitopes or fragments thereof). The term "antigen-binding site" herein refers to a three-dimensional spatial site recognized by an antibody or an antigen-binding fragment described herein.

"Epitope" refers to a site on an antigen to which an immunoglobulin or an antibody specifically binds. Epitopes can be formed with adjacent amino acids or formed by tertiary folding of non-adjacent amino acids. An epitope formed from contiguous amino acids is generally retained after exposure to a denaturing solvent, while an epitope formed by tertiary folding is generally lost after a denaturing solvent treatment. An epitope generally comprises, for example, at least 3-15 amino acids in a unique spatial conformation. Methods for determining what epitope is bound by a given antibody are well known in the art and include an immunoblotting assay, an immunoprecipitation assay, and the like. Methods for determining the spatial conformation of an epitope include techniques in the art and techniques described herein, such as X-ray crystallography and two-dimensional nuclear magnetic resonance.

"Specific binding", "selective binding", "selectively bind to" and "specifically bind to" refer to the binding of an antibody to an epitope on a predetermined antigen. Typically, an antibody binds to a predetermined antigen with an equilibrium dissociation constant (KD) of about less than 10⁻⁷ M or even less and with an affinity that is at least twice as high as its affinity for binding to a non-specific antigen other than the predetermined antigen or a closely related antigen (e.g., BSA, etc.), when determined by surface plasmon resonance (SPR) techniques in an instrument using recombinant human IL-4R as the analyte and an antibody as the ligand. The term "antigen-recognizing antibody" is used interchangeably herein with the term "specifically binding antibody".

"Cross-react" refers to the ability of an antibody described herein to bind to IL-4R from a different species. For example, an antibody described herein that binds to human IL-4R may also bind to IL-4R of another species. Cross-reactivity is measured by detecting specific reactivity with purified antigen in binding assays (e.g., SPR and ELISA) or binding or functional interactions with cells physiologically expressing IL-4R. Methods for determining cross-reactivity include standard binding assays as described herein, for example, surface plasmon resonance (SPR) analysis or flow cytometry.

"Neutralizing" or "blocking" antibody refers to an antibody whose binding to hIL-4R results in inhibition of biological activity of hIL-4 and/or hIL-13. Such inhibition of biological activity of hIL-4 and/or IL-13 can be assessed by measuring one or more indexes of biological activity of hIL-4 and/or hIL-13 well known in the art, such as hlL-4 and/or hIL-13-induced cell activation and binding of hEL-4 to hIL-4R. See, for example, CN103739711A. "Inhibition of growth" (e.g., involving cells) is intended to include any measurable reduction in cell growth.

"Inducing immune response" and "enhancing immune response" can be used interchangeably and refer to the stimulation (i.e., passive or adaptive) of an immune response to a particular antigen. The term "induce" specific for inducing CDC or ADCC refers to stimulating specific direct cell killing mechanism.

"Antibody-dependent cell-mediated cytotoxicity (ADCC)" means that the Fc receptor-expressing cells directly kill antibody-coated target cells by recognition of the Fc segment of the antibody. The ADCC effector function of the antibody may be reduced or eliminated by modification of the Fc segment of the IgG. The modification refers to a mutation in the heavy chain constant region of the antibody, such as a mutation selected from the group consisting of N297A, L234A and L235A of IgG1; IgG2/4 chimera, F235E of IgG4, and L234A/E235A mutation.

The fusion protein is a protein product co-expressed by two genes and obtained by DNA recombination. For example, the anti-IL-4R antibody-PE38KDEL fusion protein is a fusion protein obtained by co-expressing the anti-IL-4R antibody and the toxin molecule PE38KDEL by DNA recombination. Methods for producing and purifying antibodies and antigen-binding fragments are well known in the prior art and can be found in, for example, "Antibodies: A Laboratory Manual", Cold Spring Harbor Press (chapters 5-8 and 15). For example, mice can be immunized with human IL-4R or a fragment thereof, and the resulting antibodies can be renatured and purified, and amino acid sequencing can be performed by a conventional method. Likewise, antigen-binding fragments can be prepared by conventional methods. The antibody or the antigen-binding fragment described herein is genetically engineered to contain one or more additional human FRs in the non-human-derived CDRs. Human FR germline sequences can be obtained from the website of ImMunoGeneTics (IMGT) or from the Immunoglobulin Journal, 2001ISBN012441351.

The engineered antibody or antigen-binding fragment can be prepared and purified using conventional methods. As an example, cDNA sequences encoding the heavy and light chains can be cloned and recombined into a GS expression vector. Recombinant immunoglobulin expression vectors can be stably transfected into CHO cells. The sequence of the humanized antibody described herein was inserted into a corresponding expression vector by using a molecular cloning technique, and the corresponding humanized antibody could be obtained by using an HEK293 cell expression system for expression and production. As an example, mammalian expression systems may result in glycosylation of antibodies, particularly at the highly conserved N-terminus of the Fc region. Stable clones are obtained by expression of antibodies specifically binding to the human-derived antigen. Positive clones are expanded in a serum-free medium of a bioreactor to produce antibodies. The culture medium with the secreted antibody can be purified and collected by conventional techniques. The antibody can be filtered and concentrated by conventional methods. Soluble mixtures and polymers can also be removed by conventional methods, such as molecular sieves and ion exchange. The resulting product needs to be immediately frozen, e.g., at -70 °C, or lyophilized.

The antibody may be a monoclonal antibody (mAb), which refers to an antibody derived from a single clonal cell strain, which is not limited to eukaryotic, prokaryotic, or phage clonal cell strains. The monoclonal antibody or the antigen-binding fragment can be obtained by, for example, hybridoma technology, recombinant technology, phage display technology, synthetic technology (e.g., CDR-grafting), or other technologies known in the art.

The antibody may be a monospecific, bispecific or multispecific antibody. Multispecific antibodies may show specificity to different epitopes of a target peptide, or may also comprise antigen-binding domains that show specificity to one or more target peptides. The human anti-IL-4R antibody may be linked to, or co-expressed with, another functional molecule (such as another peptide or protein). For example, the antibody or the fragment thereof can be functionally linked (for example, by chemical coupling, genetic fusion or non-covalent binding, or in other ways) to one or more additional molecules (for example, another antibody or antigen-binding fragment) to produce a bispecific or multispecific antibody having at least one binding specificity.

"Administering", "giving" and "treating", when applied to animals, humans, experimental subjects, cells, tissues, organs or biological fluid, refer to contacting an exogenous drug, a therapeutic agent, a diagnostic agent or composition with the animals, humans, subjects, cells, tissues, organs or biological fluid. "Administering", "giving" and "treating" can refer to, for example, therapeutic, pharmacokinetic, diagnostic, research and experimental methods. The treatment of cells comprises making the reagent in contact with the cells and making the reagent in contact with fluid, where the fluid is in contact with the cells. "Administering", "giving" and "treating" also mean *in vitro* and *ex vivo* treatments, e.g. of a cell, by a reagent, diagnosis, a compound, or by another cell. "Treating", when applied to humans, veterinary or research subjects, refers to therapeutic treatment, preventive or prophylactic measures, and research and diagnostic applications. "Treatment" means to administer a therapeutic agent, such as any one of the complexes or compositions herein, internally or externally to a patient having (suspected of having or susceptible to) one or more disease symptoms for which the agent has therapeutic activity. Generally, the therapeutic agent is administered in an amount effective to alleviate one or more symptoms of the disease in the subject or population being treated, whether by inducing regression of such symptoms or inhibiting the development of such symptoms into any clinically measurable degree. The amount of therapeutic agent effective to alleviate any particular symptom of the disease (also referred to as the "therapeutically effective amount") may vary depending on factors such as the disease state, age and weight of the subject, and the ability of the drug to produce a desired therapeutic effect in the subject. Whether a symptom of a disease has been alleviated can be evaluated by any clinical testing methods commonly used by doctors or other health care professionals to evaluate the severity or progression of the symptom. Although the embodiments of the present disclosure (e.g., treatment methods or articles of manufacture) may be ineffective in alleviating the symptoms of the disease of interest in a single subject, they shall alleviate the symptoms of the disease of interest in a statistically significant number of subjects as determined by any statistical test method known in the art, such as the Student's t-test, chi-square test, U-test by Mann and Whitney, Kruskal-Wallis test (H-test), Jonckheere-Terpstra test and Wilcoxon test.

The term "naturally-occurring" as applied to an object refers to the fact that the object can be found in nature. For example, a polypeptide or polynucleotide sequence that is present in an organism (including viruses) that can be isolated from a source in nature and which has not been intentionally modified by man is naturally-occurring.

"Effective amount" comprises an amount sufficient to ameliorate or prevent a symptom or sign of a medical condition. An effective amount also refers to an amount sufficient to allow or facilitate diagnosis. The effective amount for a particular subject or veterinary subject may vary depending on the factors such as the disorder to be treated, the general health of the subject, the method and route and dosage of administration, and the severity of side effects. An effective amount may be the maximum dose or administration regimen to avoid significant side effects or toxic effects.

"Exogenous" refers to substances that are produced outside an organism, cell or human body, depending on the context.

"Endogenous" refers to substances that are produced within an organism, cell or human body, depending on the context.

"Homology" or "identity" refers to sequence similarity between two polynucleotide sequences or between two polypeptides. When positions in two compared sequences are occupied by identical bases or amino acid monomer subunits, e.g., if the position of each of two DNA molecules is occupied by adenine, the molecules are homologous at that position. The homology percentage between two sequences is a function of the number of matching or homologous positions shared by the two sequences divided by the number of positions compared × 100%. For example, if 6 out of 10 positions are matched or homologous when two sequences are optimally aligned, the two sequences are 60% homologous. Generally, when two sequences are aligned, comparison is performed to obtain the maximum homology percentage. The "at least 85% sequence identity" described herein means that when the variant and the parent sequence are aligned, the two sequences are at least 85% homologous; in some embodiments, they are at least 86%, 87%, 88%, 89%, 90%, 91%, 92%, 93%, 94%, 95%, 96%, 97%, 98% or 99% homologous; in some specific embodiments, they are 90%, 95% or 99% or more homologous; in other specific embodiments, they are at least 95% homologous. The amino acid sequence having at least 85% sequence identity is obtained by one or more amino acid deletion, insertion or substitution mutations made in the parent sequence.

As used herein, the terms "cell", "cell line", "cell strain" and "cell culture" are used interchangeably, and all such terms include their progeny. Therefore, the terms "transformant" and "transformed cell" include primary test cells and cultures derived therefrom, regardless of the number of times passaging is performed. It should also be understood that progeny and the parent cell may not be precisely identical in DNA content due to deliberate or inadvertent mutations. The term includes mutant progeny that have the same function or biological activity as the parent cell obtained by screening from the originally transformed cells are included.

"Optional" or "optionally" means that the event or circumstance subsequently described may, but does not necessarily, occur, and that the description includes instances where the event or circumstance occurs or does not occur. For example, "optionally comprising 1-3 antibody heavy chain variable regions" means that the antibody heavy chain variable region may, but does not necessarily, exist.

The term "vector" refers to a nucleic acid molecule capable of transporting another nucleic acid to which it has been linked. In one embodiment, the vector is a "plasmid" that refers to a circular double-stranded DNA loop into which additional DNA segments may be ligated. In another embodiment, the vector is a viral vector, wherein additional DNA segments may be ligated into the viral genome. The vectors disclosed herein are capable of autonomous replication in a host cell into which they have been introduced (e.g., bacterial vectors having a bacterial origin of replication and episomal mammalian vectors) or being integrated into the genome of a host cell upon introduction into the host cell and thereby replicated along with the host genome (e.g., non-episomal mammalian vectors).

The term "pharmaceutical composition" refers to a mixture of one or more of the complexes or the physiologically/pharmaceutically acceptable salts or prodrugs thereof described herein, and other components, wherein the other components are, for example, physiologically/pharmaceutically acceptable vehicles and excipients. The pharmaceutical composition is intended to promote the administration to an organism, so as to facilitate the absorption of the active ingredient, thereby exerting biological activity.

The term "excipient" is an addition, besides the active ingredient, to a pharmaceutical formulation. It may also be referred to as an auxiliary material. For example, binders, fillers, disintegrants, lubricants in tablets; the matrix part in semisolid ointment and cream preparations; preservatives, antioxidants, corrigents, fragrances, cosolvents, emulsifiers, solubilizers, osmotic pressure regulators, colorants and the like in liquid formulations can all be referred to as excipients.

The term "diluent", also referred to as filler, is used primarily to increase the weight and volume of the tablet. The addition of the diluent not only ensures a certain volume, but also reduces the dose deviation of the main ingredients, and improves the drug's compression moldability and the like. When the drug in the tablet form contains oily components, an absorbent is necessarily added to absorb the oily components so as to maintain a "dry" state and thus to facilitate the preparation of the tablet. Examples include starch, lactose, inorganic salts of calcium, microcrystalline cellulose and the like.

"Immunohistochemical scoring" is a histological scoring method for processing immunohistochemical results, wherein the number of positive cells in each section and the staining intensity thereof are converted into corresponding numerical values so as to achieve the aim of semi-quantifying tissue staining. A score of 0 indicates no staining observed or indicates that membrane staining is observed in less than 10% of tumor cells; a score of +1 indicates that slightly/barely detectable membrane staining is detected in more than 10% of tumor cells; a score of +2 indicates that moderate complete membrane staining is observed in more than 10% of tumor cells; a score of +3 indicates that intense complete membrane staining is observed in more than 10% of tumor cells. In the present disclosure, those samples with an IL-4R expression score of 0 or +1 can be considered to not overexpress IL-4R, while those with a score of +2 or +3 can be considered to overexpress IL-4R.

The present application is further described below with examples; however, these examples are not intended to limit the scope of protection.

Experimental procedures without specific conditions indicated in the examples or test examples are generally conducted according to conventional conditions, or according to conditions recommended by the manufacturers of the starting materials or commercial products. See Sambrook et al., Molecular Cloning, Cold Spring Harbor Laboratory; and Current Protocols in Molecular Biology, Ausubel et al., Greene Publishing Association, Wiley Interscience, NY Reagents without specific origins indicated are commercially available conventional reagents.

### Example 1. Preparation of Exemplary Immunotoxins

The single-chain variable fragment (scFv) of the humanized IL-4R monoclonal antibody hu25G7 (whose heavy chain variable region sequence is set forth in SEQ ID NO: 43 as hu25G7-VH and whose light chain variable region sequence is set forth in SEQ ID NO: 37 as hu25G7-A LCVR) was fused and expressed with mutated and truncated pseudomonas exotoxin (PE38KDEL) (SEQ ID NO: 49) via a linker ASGGPE (SEQ ID NO: 50), wherein the amino acids at positions VH-44 and VL-100 of the hu25G7 antibody were mutated into cysteine (as set forth in SEQ ID NOs: 47-48), and VH and VL were linked by a linking peptide (GGGGS)₃ (SEQ ID NO: 51); the IL-4R-targeted immunotoxin 25G7-(scdsFv)-PE38KDEL (hereinafter referred to as 25G7-IT) was thus obtained, the complete sequence being set forth in SEQ ID NO: 52.

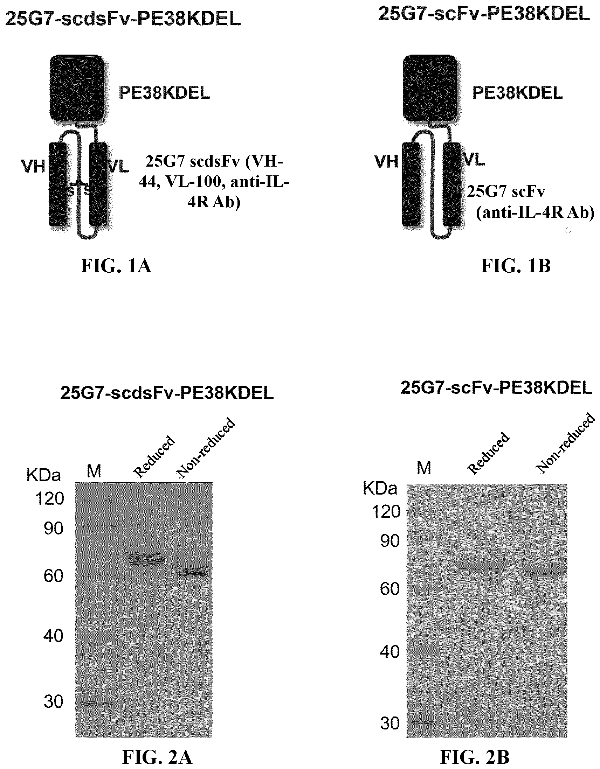

FIG. 1A and FIG. 1B are schematic diagrams of the structures of two immunotoxins. 25G7-IT was expressed in *E. coli* BL21, and protein purification and renaturation were performed from inclusion bodies; then, endotoxin was removed to obtain purified 25G7-IT with a molecular weight of about 63 KDa (SDS-PAGE results are shown in FIG. 2A and FIG. 2B).

The heavy chain sequence of the humanized anti-human IL-4R antibody 25G7 is set forth in SEQ ID NO: 17, and the light chain sequence is set forth in SEQ ID NO: 18. Antibody expression and purification can be performed according to the conventional methods for antibody expression and purification in the art, e.g., the methods described in the patent application WO2020038454A1. The foregoing patent application is incorporated herein by reference in its entirety.
**Sequence of hu25G7-VH-44** 
EVQLVESGGGLVQPGGSLRLSCAASGFTFSDYGMHWVRQAPGKCLEWVAFISS
GSSIIYYADIVKGRSTISRDNAKNTLYLQMNSLRAEDTAVYYCTRGNKRGFFDY
WGQGTLVTVSS SEQ ID NO: 47;
**Sequence of hu25G7-VL-100** 
EIVLTQSPATLSLSPGERATLSCRASSSVPYMYWYQQKPGQAPRLLIYLTSNLAS
GIPARFSGSGSGTDFTLTISSLEPEDFAVYYCQQWRAYPPMLTFGCGTKVEIK
SEQ ID NO: 48;
**Sequence of PE38KDEL:** 
EGGSLAALTAHQACHLPLETFTRHRQPRGWEQLEQCGYPVQRLVALYLAARLS
WNQVDQVIRNALASPGSGGDLGEAIREQPEQARLALTLAAAESERFVRQGTGN
DEAGAANGPADSGDALLERNYPTGAEFLGDGGDVSFSTRGTQNWTVERLLQA
HRQLEERGYVFVGYHGTFLEAAQSIVFGGVRARSQDLDAIWRGFYIAGDPALA
YGYAQDQEPDARGRIRNGALLRVYVPRSSLPGFYRTSLTLAAPEAAGEVERLIG
HPLPLRLDAITGPEEEGGRLETILGWPLAERTVVIPSAIPTDPRNVGGDLDPSSIPD
KEQAISALPDYASQPGKPPKDEL SEQ ID NO: 49;
**Sequence of linker L1:**
   ASGGPE SEQ ID NO: 50;
**Sequence of linking peptide L2:**
   GGGGSGGGGSGGGGS SEQ ID NO: 51;
**Sequence of 25G7-(scdsFv)-PE38KDEL (25G7-IT):**

EVQLVESGGGLVQPGGSLRLSCAASGFTFSDYGMHWVRQAPGKCLEWVAFISS
GSSIIYYADIVKGRSTISRDNAKNTLYLQMNSLRAEDTAVYYCTRGNKRGFFDY
WGQGTLVTVSSGGGGSGGGGSGGGGSEIVLTQSPATLSLSPGERATLSCRASSSV
PYMYWYQQKPGQAPRLLIYLTSNLASGIPARFSGSGSGTDFTLTISSLEPEDFAVY
YCQQWRAYPPMLTFGCGTKVEIKASGGPEEGGSLAALTAHQACHLPLETFTRHR
QPRGWEQLEQCGYPVQRLVALYLAARLSWNQVDQVIRNALASPGSGGDLGEAI
REQPEQARLALTLAAAESERFVRQGTGNDEAGAANGPADSGDALLERNYPTGA
EFLGDGGDVSFSTRGTQNWTVERLLQAHRQLEERGYVFVGYHGTFLEAAQSIV
FGGVRARSQDLDAIWRGFYIAGDPALAYGYAQDQEPDARGRIRNGALLRVYVP
RSSLPGFYRTSLTLAAPEAAGEVERLIGHPLPLRLDAITGPEEEGGRLETILGWPL

AERTVVIPSAIPTDPRNVGGDLDPSSIPDKEQAISALPDYASQPGKPPKDEL
SEQ ID NO: 52.

In addition, we have developed an immunotoxin fusion protein 25G7-(scFv)-PE38KDEL, a format without introduction of additional interchain disulfide bonds, the amino acid sequence of which is set forth in SEQ ID NO: 53.
**Sequence of 25G7-(scFv)-PE38KDEL:**

EVQLVESGGGLVQPGGSLRLSCAASGFTFSDYGMHWVRQAPGKGLEWVAFISS
GSSIIYYADIVKGRSTISRDNAKNTLYLQMNSLRAEDTAVYYCTRGNKRGFFDY
WGQGTLVTVSSGGGGSGGGGSGGGGSEIVLTQSPATLSLSPGERATLSCRASSSV
PYMYWYQQKPGQAPRLLIYLTSNLASGIPARFSGSGSGTDFTLTISSLEPEDFAVY
YCQQWRAYPPMLTFGGGTKVEIKASGGPEEGGSLAALTAHQACHLPLETFTRH
RQPRGWEQLEQCGYPVQRLVALYLAARLSWNQVDQVIRNALASPGSGGDLGE
AIREQPEQARLALTLAAAESERFVRQGTGNDEAGAANGPADSGDALLERNYPT
GAEFLGDGGDVSFSTRGTQNWTVERLLQAHRQLEERGYVFVGYHGTFLEAAQ
SIVFGGVRARSQDLDAIWRGFYIAGDPALAYGYAQDQEPDARGRIRNGALLRVY
VPRSSLPGFYRTSLTLAAPEAAGEVERLIGHPLPLRLDAITGPEEEGGRLETILGW
PLAERTVVIPSAIPTDPRNVGGDLDPSSIPDKEQAISALPDYASQPGKPPKDEL
SEQ ID NO: 53.

### Example 2. Determination of Affinity Kinetics of Interactions

The affinity kinetics of the interactions of 25G7-IT and the reference sample MDNA55 (expressed in an *E. coli* system and purified) with hrIL-4R-Fc (II,R-H5253, Aero) was determined on Biacore 8K (GE) using the surface plasmon resonance (SPR) technique. This experiment used the capture method: an anti-human IgG antibody (29234600, GE) was covalently coupled onto a CM5 chip (29149603, GE), rhIL-4R-Fc was then captured as a ligand on the chip, 25G7-IT and the reference sample MDNA55 were then each injected as analytes, and affinity analysis and calculation were performed.

The experimental results show that 25G7-IT and the reference sample MDNA55 have strong affinities for rhIL-4R-Fc, with KD of 1.01E-10 and 4.39E-10 M, respectively (Table 2). The affinity of 25G7-IT for rhIL-4R-Fc is significantly greater than that of the reference sample MDNA55 for rhIL-4R-Fc.

**Table 2. The kinetic affinities of complexes determined by Biacore**

| **Ligand** | **Test sample** | **ka (1/Ms)** | **kd (1/s)** | **KD (M)** |
|---|---|---|---|---|
| rh IL-4R-Fc | 25G7-scdsFv-PE38KDEL | 2.77E+06 | 2.79E-04 | 1.01E-10 |
| | MDNA55 | 6.62E+06 | 2.90E-03 | 4.39E-10 |

### Example 3. Determination of Binding Capacity to GBM Target Cells

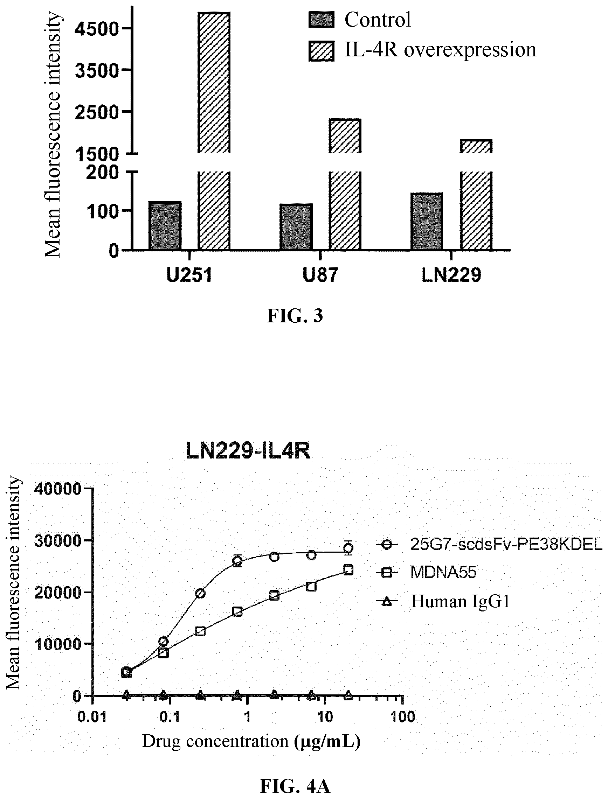

The endogenous IL-4R expression in the GBM cell lines U87, U251 and LN229 is low. Therefore, the lentivirus system was used to overexpress human IL-4R in these three GBM cell lines. After G418 screening, the effect of IL-4R overexpression was identified by flow cytometry (FIG. 3). The results show that IL-4R was stably overexpressed in these three cell lines.

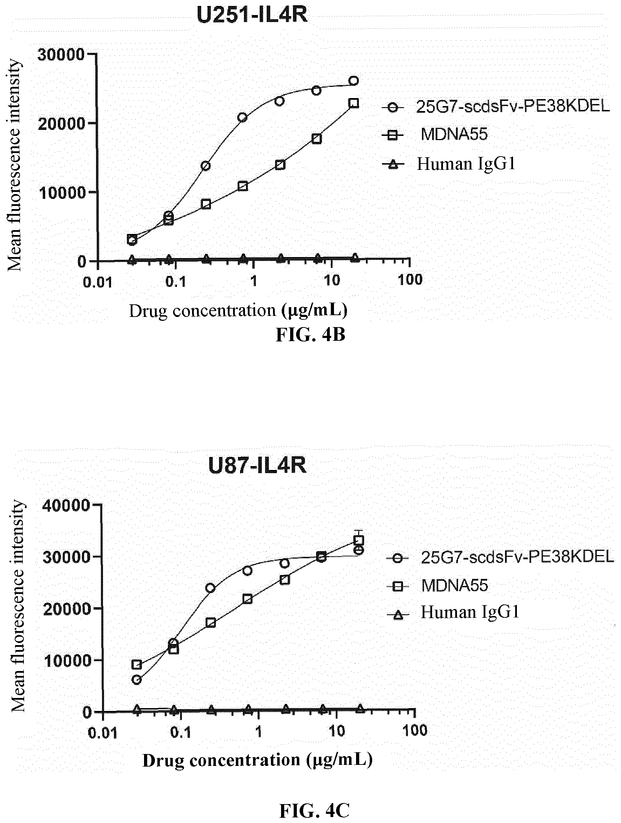

Complete medium was prepared by adding 10% FBS and 1% penicillin/streptomycin to DMEM, and the U87-, U251- and LN229-IL4R stably transfected cell strains were cultured in a 37 °C incubator containing 5% CO₂. Cells in T75 flasks were digested with 1.5 mL of TrpLE (Gibco, #12605-010) at 37 °C for 3-5 min and then neutralized with DMEM containing FBS. After centrifugation, cell suspensions with a concentration of 2 × 10⁶ cells/mL were prepared and added to a 96-well round-bottom plate at 50 µL/well. 25G7-IT, MDNA55 and a hIgG1 isotype control were each diluted with DMEM to 8 concentration points (three-fold gradient: 20; 6.67; 2.22; 0.74; 0.25; 0.08; 0.03; 0 µg/mL), and were added to the 96-well plate at 50 µL/well and well mixed with the cells, and the complexes and the cells were co-incubated on ice for 40 min. After the incubation was complete, the cells were washed three times with 200 µL of flow cytometry buffer. An anti-PE38 antibody (Sigma-aldrich, Cat: P2318-1ML) was 1:300 diluted with flow cytometry buffer and added to the 96-well plate at 50 µL/well to resuspend the cells, and the antibody and the cells were co-incubated on ice for 40 min. The cells were then washed three times with 200 µL of flow cytometry buffer. Anti-rabbit IgG Fab2 Alexa Fluor 647 (Cell Signaling Technology, #02/2020) was 1:500 diluted with flow cytometry buffer and added to the 96-well plate at 50 µL/well to resuspend the cells, and the antibody and the cells were co-incubated on ice for 30 min. After centrifugation and three washes with 200 µL of flow cytometry buffer, the cells were resuspended, and the single-cell Alexa Fluor647 signal in each well was detected by BD FACSCelestaTM flow cytometer. The mean fluorescence intensity (MFI) was analyzed, and the experimental results are shown in Table 3 and FIG. 4A to FIG. 4C: both 25G7-IT and the reference sample MDNA55 can bind to IL-4R-positive GBM cell lines, and the binding affinity of 25G7-IT for the U87-, U251- and LN229-IL4R stably transfected cell strains is greater than that of MDNA55.

**Table 3. The binding capacity results of complexes to GBM target cells**

| LN229-IL4R | Mean MFI | | |
|---|---|---|---|
| Concentration (µg/mL) | 25G7-scdsFv-PE38KDEL | MDNA55 | Human IgG1 |
| 20 | 28521.5 | 24309 | 231 |
| 6.666667 | 27154 | 21148 | 244.5 |
| 2.222222 | 26803.5 | 19382 | 361 |
| 0.740741 | 26046 | 16242 | 286.5 |
| 0.246914 | 19769 | 12443 | 300 |
| 0.082305 | 10449.5 | 8311 | 318.5 |
| 0.027435 | 4750.5 | 4474.5 | 352.5 |
| 0 | 498 | | |

| U87-IL4R | Mean MFI | | |
|---|---|---|---|
| Concentration (µg/mL) | 25G7-scdsFv-PE38KDEL | MDNA55 | Human IgG1 |
| 20 | 30861 | 32745.5 | 317.5 |
| 6.666667 | 29485.5 | 29702 | 306.5 |
| 2.222222 | 28386 | 25262 | 334.5 |
| 0.740741 | 27025 | 21574 | 339 |
| 0.246914 | 23766.5 | 17098 | 321.5 |
| 0.082305 | 13190.5 | 12020.5 | 393.5 |
| 0.027435 | 6168 | 9121 | 588.5 |
| 0 | 426 | | |

| U251-IL4R | Mean MFI | | |
|---|---|---|---|
| Concentration (µg/mL) | 25G7-scdsFv-PE38KDEL | MDNA55 | Human IgG1 |
| 20 | 25827 | 22597 | 275 |
| 6.666667 | 24461 | 17477 | 288 |
| 2.222222 | 22952 | 13736 | 282 |
| 0.740741 | 20635 | 10682 | 284 |
| 0.246914 | 13672 | 8131 | 287 |
| 0.082305 | 6511 | 5860 | 274 |
| 0.027435 | 2947 | 3179 | 263 |
| 0 | 237 | | |

### Example 4. Determination of Endocytosis Efficiencies in GBM Target Cells

To determine the endocytosis efficiencies of 25G7-IT and the reference sample MDNA55 in GBM target cells, the endocytosis efficiencies of 25G7-IT and MDNA55 in the three strains of cerebral glioma cells stably transfected with IL-4R described above were compared.

According to the experimental results in Example 3, the final concentration of 2 µg/mL was selected as a concentration for endocytosis efficiency determination as the endocytosis efficiency in the three cell samples was substantially saturated at that concentration. 25G7-IT, MDNA55 and a hIgG1 isotype control were diluted with DMEM to 2 µg/mL, added to a 96-well plate at 50 µL/well and well mixed with cells, and the complexes and the cells were co-incubated on ice for 40 min. After three washes with DMEM complete medium, the cells were resuspended in 100 µL of DMEM complete medium. 100 µL of the cells labeled with 0 h in the 96-well plate was transferred to another 96-well round-bottom plate, and 100 µL, of 4% paraformaldehyde fixative solution was added; the cells were fixed at 4 °C. After the remaining cells were cultured in a 37 °C incubator for 1 h, 2 h, 4 h and 24 h, the corresponding cells were taken out, transferred to another 96-well plate and fixed at 4 °C in the same manner.

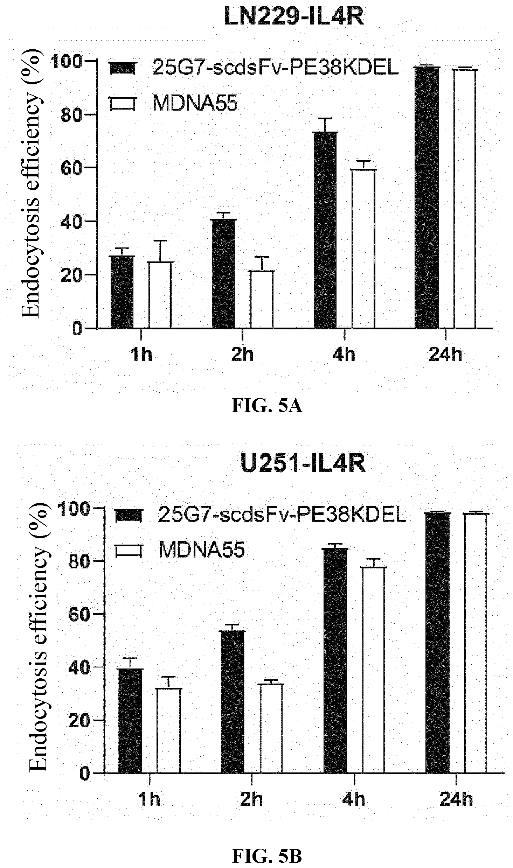

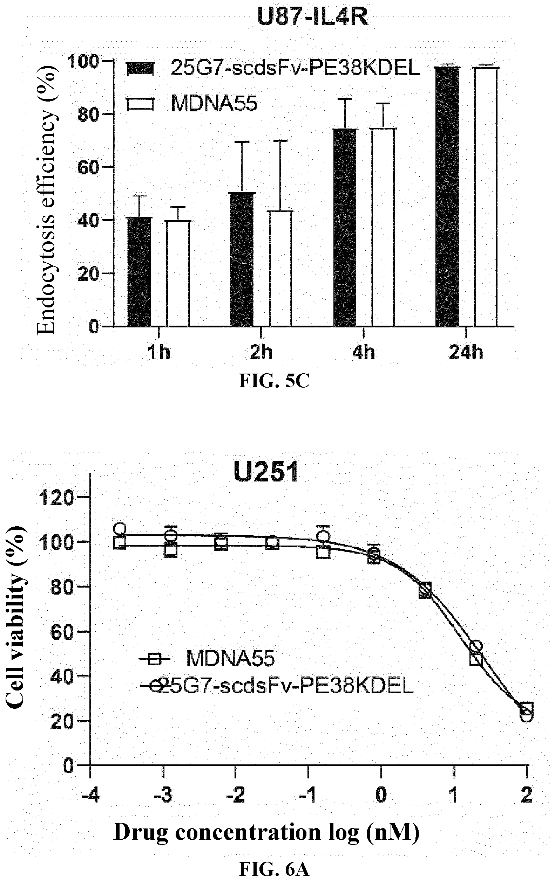

Cells of 0-h to 4-h endocytosis were subjected to flow cytometry antibody staining on the same day; cells after 24-h endocytosis was subjected to flow cytometry antibody staining and flow cytometry analysis when incubated until the end of the experiment. When the cells of 4-h endocytosis were fixed for half an hour, all of the fixed cells were centrifuged and washed twice with flow cytometry buffer. An anti-PE38 antibody (Sigma-aldrich, Cat: P2318-1ML) was 1:300 diluted with flow cytometry buffer and added to the 96-well plate at 50 µL/well to resuspend the cells, and the antibody and the cells were then co-incubated on ice for 40 min. The subsequent experimental steps were the same as those in Example 3, and the analysis results are shown in Table 4 and FIG. 5A to FIG. 5C. The experimental results show that 25G7-IT and both the reference sample MDNA55 can be effectively endocytosed in IL-4R-overexpressing GBM cells; the endocytosis efficiency in the three types of cells at 4 h reached 60% to 80%, and the endocytosis efficiency of 25G7-IT in the LN229- and U251-IL4R stably transfected cell strains is greater than that of MDNA55.

**Table 4. The endocytosis efficiency results in GBM target cells**

| | Mean endocytosis efficiency (%) | |
|---|---|---|
| LN229-IL4R | 225 G7-scdsFv-PE3 8KDEL | MDNA55 |
| 0h | 0 | 0 |
| 1h | 40.005 | 32.765 |
| 2h | 54.405 | 34.3 |
| 4h | 85.25 | 78.4 |
| 24h | 98.83 | 98.57 |

| U87-IL4R | 25 G7-scdsFv-PE3 8KDEL | MDNA55 |
|---|---|---|
| 0h | 0 | 0 |
| 1h | 41.81 | 40.52 |
| 2h | 51.155 | 44.06 |
| 4h | 75.145 | 75.41 |
| 24h | 98.25 | 98.18 |

| U251-IL4R | 25 G7-scdsFv-PE3 8KDEL | MDNA55 |
|---|---|---|
| 0h | 0 | 0 |
| 1h | 27.72 | 25.335 |
| 2h | 41.515 | 21.96 |
| 4h | 73.98 | 60.015 |
| 24h | 98.295 | 97.43 |

### Example 5. Determination of In Vitro Killing Efficiencies in GBM Target Cells

To determine the *in vitro* killing efficiencies of 25G7-IT and the reference sample MDNA55 in GBM target cells, the CTG (CELL TITER-GLO) method was used to compare the *in vitro* efficacy of the two in the three strains of brain tumor cells stably transfected with human IL-4R.

10% FBS and 1% penicillin/streptomycin were added to DMEM to prepare complete medium. In a 37 °C incubator containing 5% CO₂, the U87-IL4R, U251-IL4R and LN229-IL4R stably transfected cell strains and the U87, U251 and LN229 control cells were cultured.

Plating was performed in a 96-well plate at 4000 cells and 130 µL, per well. After the plate was left overnight, when cell adhesion was complete, 20 µL of gradient concentrations of drug containing 25G7-IT or MDNA55 (final concentrations: 100 nM, 20 nM, 4 nM, 0.8 nM, 0.16 nM, 0.032 nM, 0.0064 nM, 0.00128 nM, 0.000256 nM and 0 nM) was added, and the cells were cultured in an incubator for 3 days. After three days, the 96-well plate was taken out of the incubator and equilibrated to room temperature, and 50 µL of CTG reagent (CellTiter-Glo Luminescent, Promega, #G7573) was added to the cell suspensions. The plate was shaken on a shaker for 2 min to well mix the suspensions and was then left to stand at room temperature for 10 min. Cell viability was measured using the built-in Luminescence mode of EnVision2105 Multimode Plate Reader (PerkinElmer). The absorbance results were converted into percentages and then analyzed using Prism 8.

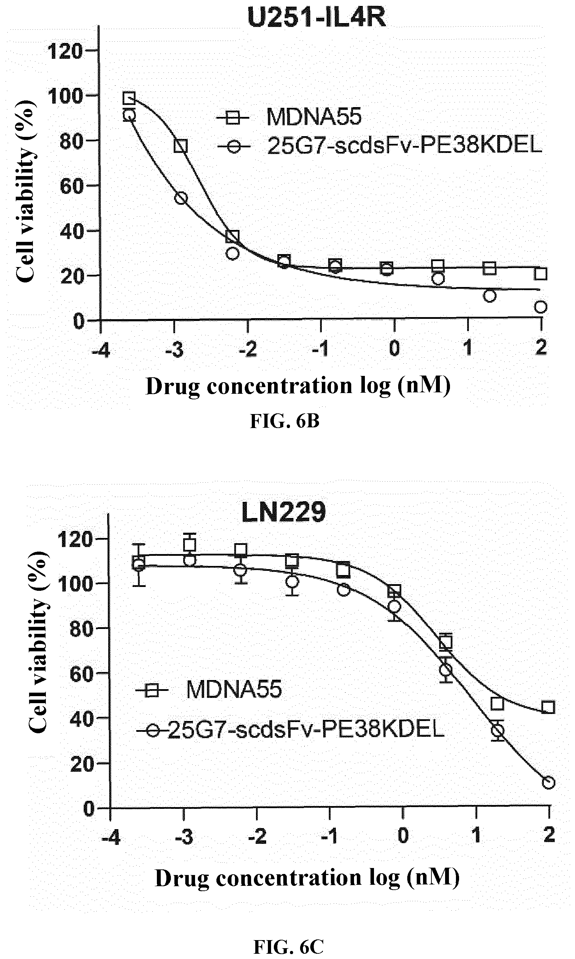

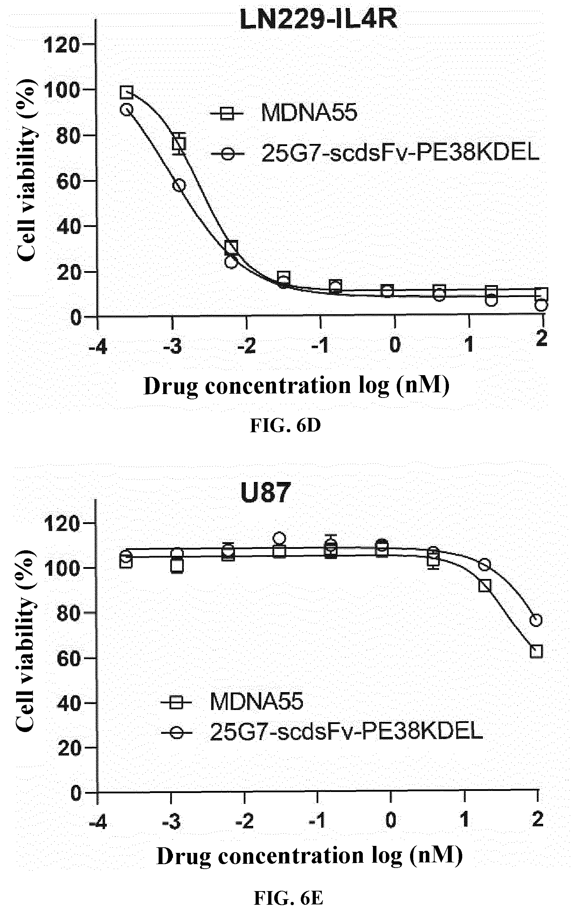

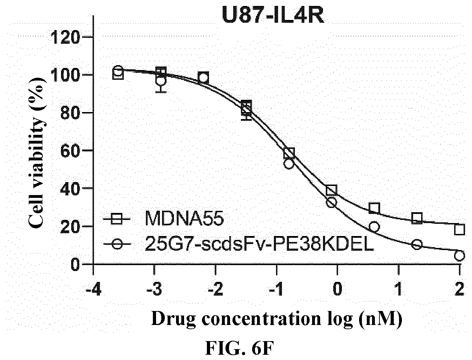

The experimental results (FIG. 6A to FIG. 6F) show that both 25G7-IT and the reference sample MDNA55 can efficiently kill the target cells among the IL-4R-overexpressing GBM cells, and particularly, the efficacy against LN229-IL4R is high: the *in vitro* killing effect reached 80% at the concentration of 0.1 nM. In general, the *in vitro* killing efficiency of 25G7-IT in the three stains of cerebral glioma cells stably transfected with IL-4R-LN229, U251 and U87-is higher than that of MDNA55.

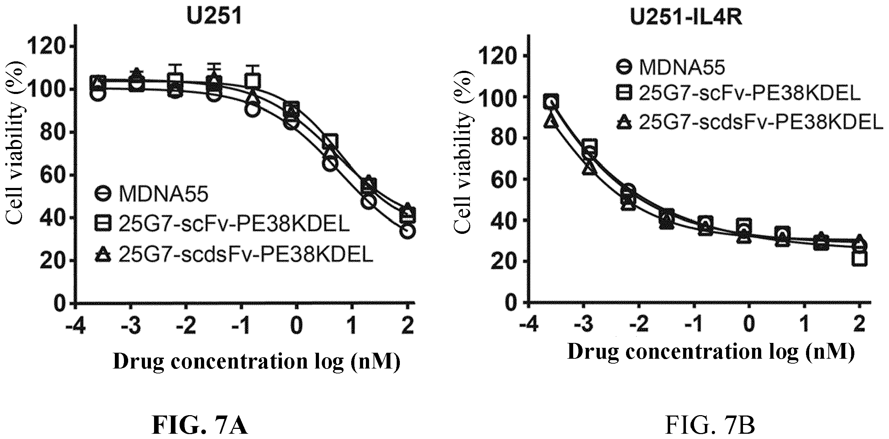

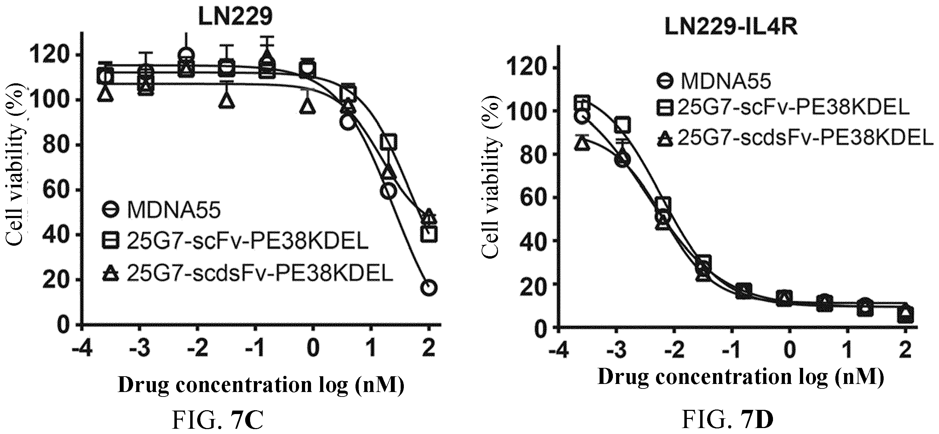

In addition, the killing efficiency of the drug 25G7-scFv-PE38KDEL, a format without the interchain disulfide bond, in the LN229 and U251 IL-4R stably transfected cerebral glioma cell strains was compared with those of 25G7-scdsFv-PE38KDEL (25G7-IT) and MDNA55. FIG. 7A to FIG. 7D show that 25G7-scFv-PE38KDEL has a similar *in vitro* killing efficiency in cerebral glioma cells to 25G7-IT and MDNA55.

### Example 6. In Vivo Efficacy of IL-4R-Targeted Drugs in GBM Tumor Model

To determine the *in vivo* efficacy of 25G7-IT and the reference sample MDNA55 in the GBM tumor model, a U87-MG subcutaneous inoculation model with 1+ (immunohistochemical score) IL-4R expression was first constructed. 2 × 10⁶ U87-MG cells resuspended in PBS and matrigel (1:1) were subcutaneously inoculated into the right side of the back of laboratory mice (0.1 mL/mouse), and tumor growth was observed regularly. When the mean size of the subcutaneous inoculated tumors reached approximately 100-150 mm³, the mice were divided into groups of 8. Multiple-site intratumoral administration of the drugs 25G7-scdsFv-PE38KDEL (25G7-IT) and MDNA55 was performed at a dose of 0.5 mg/kg (mpk) once a week, and a total of four doses were administered. After administration began, the body weight of the mice and the tumor size were measured twice a week. The calculation formula for tumor volume was: tumor volume (mm³) = 1/2 × (a × b²) (where a represents long-axis diameter and b represents short-axis diameter).

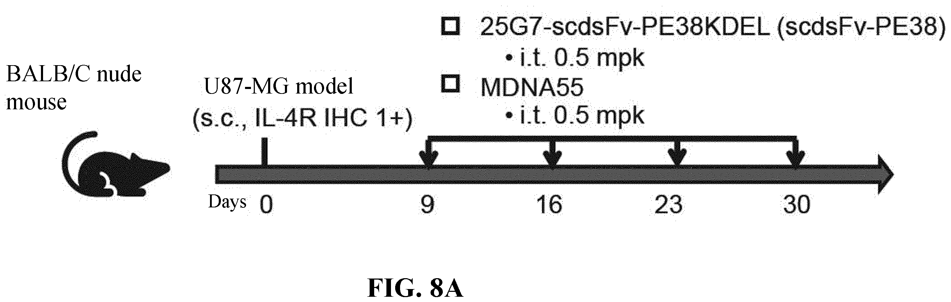

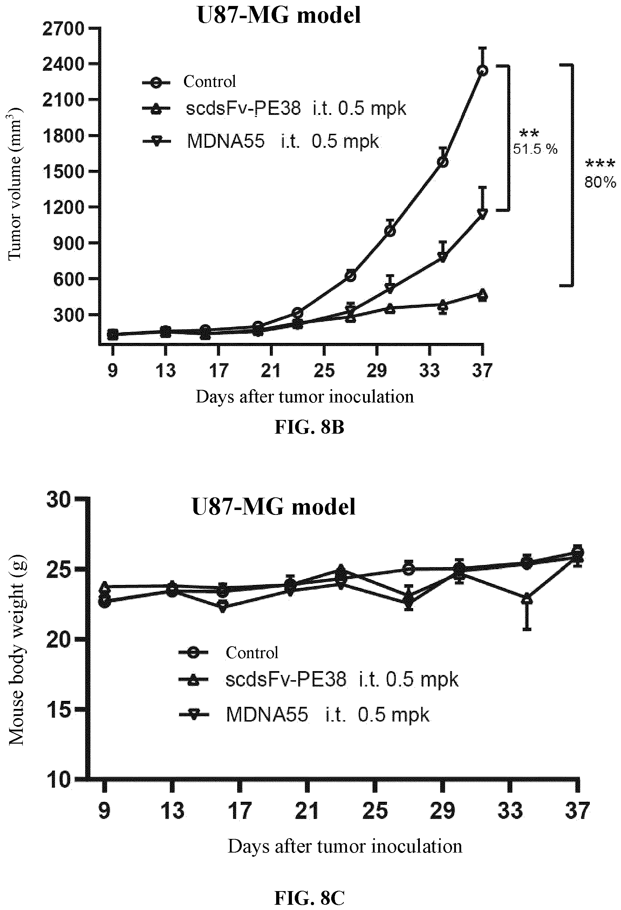

The experimental results are shown in FIG. 8A to FIG. 8C and Table 5. In the U87-MG tumor model lowly expressing IL-4R (with an immunohistochemical score of 1+), administration was performed at 0.5 mpk once a week, and after 4 doses, 25G7-IT showed 80% TGI (tumor growth inhibition), the reference sample MDNA55 showed 51% TGI. The results indicate that in the glioma model lowly expressing IL-4R, 25G7-IT had a significantly better inhibitory effect on tumor growth than MDNA55.

**Table 5. The in vivo efficacy of IL-4R-targeted drugs in the mouse glioma model**

| Inoculated cell (s.c.) | IL-4R expression | Drug | Dose (mpk, QW) | Route of administration | Number of mice # | Tumor growth inhibition (statistical difference from control) |
|---|---|---|---|---|---|---|
| U87-MG | 1+ | hIgG1 isotype control | | | 8 | |
| | | 25G7-scdsFv-PE38KDEL | 0.5 | i.t. | 8 | 80% *** |
| | | MDNA55 | 0.5 | i.t. | 8 | 51.5% ** |

| | | | | | | |
|---|---|---|---|---|---|---|
| Note: **s.c.**: subcutaneous; **mpk**: mg/kg (mg per kg body weight); **i.t**.: intratumoral injection; **i.v**.: intravenous injection; **QW**: once a week. ** indicates p < 0.01, and *** indicates p < 0.001. | | | | | | |

### Example 7. In Vivo Efficacy of IL-4R-Targeted Drugs in GBM Tumor Model (IL-4R 3+)

To further determine the *in vivo* efficacy of 25G7-IT and the reference sample MDNA55 in the GBM tumor model, the IL-4R-overexpressing LN229 cell line (denoted by LN229-IL4R, with an immunohistochemical score of 3+) was utilized to construct a subcutaneous inoculation model.

1 × 10⁷ LN229-IL4R cells resuspended in PBS (1:1) were subcutaneously inoculated into the right side of the back of laboratory mice (0.1 mL/mouse), and tumor growth was observed regularly. When the mean size of the subcutaneous inoculated tumors reached approximately 100-150 mm³, the mice were divided into groups of 6-7. MDNA55 was administered at a dose of 0.25 mpk (i.t.), and 25G7-scdsFv-PE38KDEL (25G7-IT) was administered at a dose of 0.25 mpk (i.t. or i.v.). The administration was performed once a week, and a total of four doses were administered. Tumor growth monitoring and calculation were performed as in Example 6.

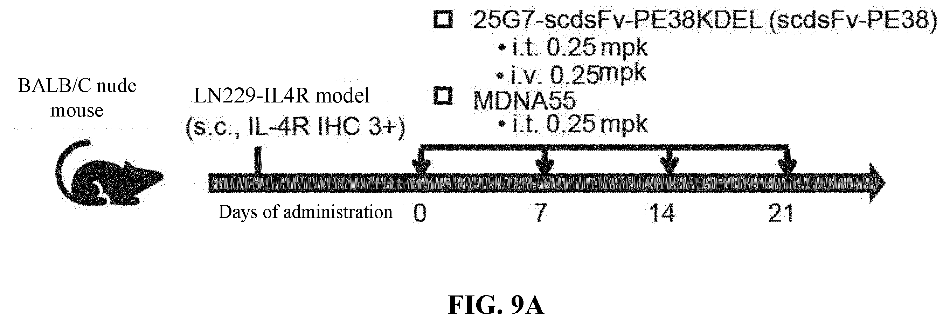

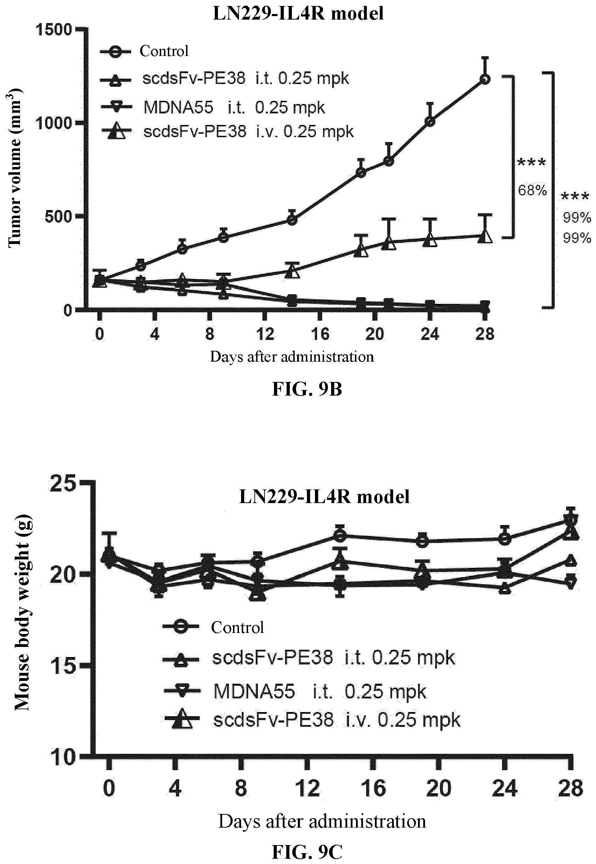

The experimental results are shown in FIG. 9A to FIG. 9C and Table 6. In the LN229-IL4R tumor model highly expressing IL-4R (with an immunohistochemical score of 3+), 25G7-IT and MDNA55 showed 99% (complete response) TGI (tumor growth inhibition) when administered at 0.25 mpk (i.t.), whereas 25G7-IT showed 68% TGI when administered at the same dose (0.25 mpk, i.v.): intratumoral administration has a better effect than intravenous administration.

In addition, in the LN229-IL4R tumor model highly expressing IL-4R (with an immunohistochemical score of 3+), the doses of immunotoxin were further reduced: 25G7-scdsFv-PE38KDEL (25G7-IT) was administered at doses of 0.1 (the second dose and the following doses were reduced to 0.05) and 0.3 (the second dose and the following doses were reduced to 0.15) mpk, and MDNA55 was administered at a dose of 0.1 (the second dose and the following doses are reduced to 0.05) mpk, intratumorally.

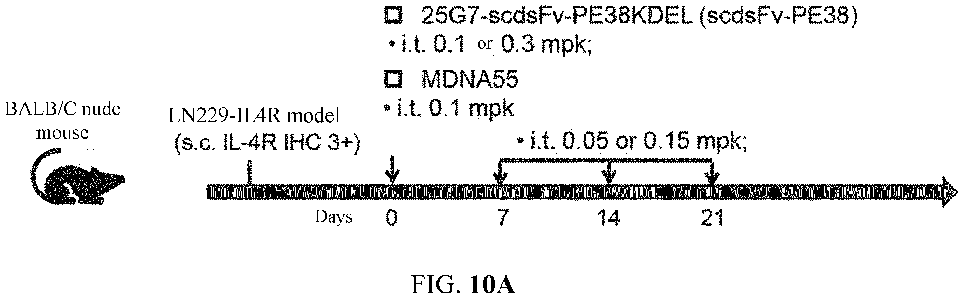

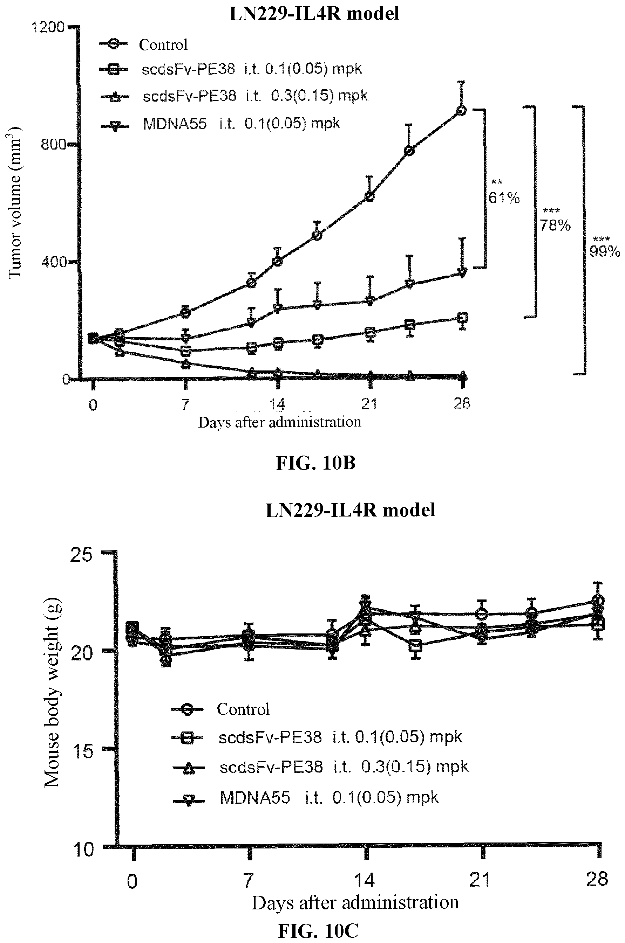

The experimental results are shown in FIG. 10A to FIG. 10C and Table 6. In the LN229-IL4R tumor model (with an immunohistochemical score of 3+), administration was performed at 0.1 (first dose) or 0.05 (the second dose to the fourth dose) mpk once a week, and after 4 doses, 25G7-IT showed 77% TGI (tumor growth inhibition), whereas the reference sample MDNA55 showed 59% TGI; and when the 25G7-IT drug was administered at a dose of 0.3 (the first dose) or 0.15 (the second dose to the fourth dose), the tumors in tumor-bearing mice achieved complete response (TGI = 99%). The results show that in the glioma model highly expressing IL-4R, the low concentration of 25G7-IT had a better inhibitory effect on tumor growth than MDNA55 at the same dose, whereas the high dose of the 25G7-IT drug could make tumor-bearing mice achieve complete response: a significant dose-effect relationship was shown.

**Table 6. The in vivo efficacy of IL-4R-targeted drugs in the mouse glioma model**

| Inoculated cell (s.c.) | IL-4R expression | Drug | Dose (mpk, QW) | Route of administration | Number of mice # | Tumor growth inhibition (statistical difference from control) |
|---|---|---|---|---|---|---|
| LN229-IL4R | 3+ | hIgG1 isotype control | | | 6 | |
| | | 25G7-scdsFv-PE38KDEL | 0.25 | i.t. | 7 | 99% *** |
| | | 25G7-scdsFv-PE38KDEL | 0.25 | i.v. | 6 | 68% *** |
| | | MDNA55 | 0.25 | i.t. | 7 | 99% *** |
| LN229-IL4R | 3+ | hIgG1 isotype control | | | 6 | |
| | | 25G7-scdsFv-PE38KDEL | 0.1 → 0.05 | i.t. | 7 | 78% *** |
| | | 25G7-scdsFv-PE38KDEL | 0.3 → 0.15 | i.t. | 7 | 99% *** |
| | | MDNA55 | 0.1 → 0.05 | i.t. | 7 | 61%** |

| | | | | | | |
|---|---|---|---|---|---|---|
| Note: **s.c.:** subcutaneous; **mpk:** mg/kg (mg per kg); **i.t.:** intratumoral injection; **i.v.:** intravenous injection; **QW:** once a week. ^{∗∗} indicates p < 0.01, and ^{∗∗∗} indicates p < 0.001. | | | | | | |

The use and welfare of the laboratory animals in the present disclosure were carried out in compliance with the provisions of Association for Assessment and Accreditation of Laboratory Animal Care, International (AAALAC). The health and death of the animals were monitored daily, and routine examinations included observation of the effects of the test substance and drug on the daily performance of the animals, such as behavioral activities, weight changes and appearance.

## Claims

1. A complex of an anti-IL-4R antibody or an antigen-binding fragment thereof, comprising:
- the anti-IL-4R antibody or the antigen-binding fragment thereof, and
- one or more toxin molecules;
wherein the anti-IL-4R antibody or the antigen-binding fragment thereof is covalently or non-covalently linked to the toxin molecules;
the anti-IL-4R antibody or the antigen-binding fragment thereof comprises any one selected from the group consisting of (I) to (IV):
(I) a heavy chain variable region comprising a HCDR1, a HCDR2 and a HCDR3 whose amino acid sequences are set forth in SEQ ID NO: 3, SEQ ID NO: 4 and SEQ ID NO: 5, respectively; and
a light chain variable region comprising a LCDR1, a LCDR2 and a LCDR3 whose amino acid sequences are set forth in SEQ ID NO: 38, SEQ ID NO: 7 and SEQ ID NO: 40, respectively;
(II) a heavy chain variable region comprising a HCDR1, a HCDR2 and a HCDR3 whose amino acid sequences are set forth in SEQ ID NO: 11, SEQ ID NO: 12 and SEQ ID NO: 13, respectively; and
a light chain variable region comprising a LCDR1, a LCDR2 and a LCDR3 whose amino acid sequences are set forth in SEQ ID NO: 14, SEQ ID NO: 15 and SEQ ID NO: 16, respectively;
(III) a heavy chain variable region comprising a HCDR1, a HCDR2 and a HCDR3 whose amino acid sequences are set forth in SEQ ID NO: 3, SEQ ID NO: 4 and SEQ ID NO: 5, respectively; and
a light chain variable region comprising a LCDR1, a LCDR2 and a LCDR3 whose amino acid sequences are set forth in SEQ ID NO: 6, SEQ ID NO: 7 and SEQ ID NO: 8, respectively;
(IV) a heavy chain variable region comprising a HCDR1, a HCDR2 and a HCDR3 whose amino acid sequences are set forth in SEQ ID NO: 3, SEQ ID NO: 4 and SEQ ID NO: 5, respectively; and
a light chain variable region comprising a LCDR1, a LCDR2 and a LCDR3 whose amino acid sequences are set forth in SEQ ID NO: 42, SEQ ID NO: 39 and SEQ ID NO: 8, respectively.

2. The complex of the anti-IL-4R antibody or the antigen-binding fragment thereof according to claim 1, wherein the anti-IL-4R antibody is selected from any one of: a murine antibody, a chimeric antibody, a fully human antibody and a humanized antibody.

3. The complex of the anti-IL-4R antibody or the antigen-binding fragment thereof according to any one of claims 1-2, wherein:
the anti-IL-4R antibody or the antigen-binding fragment thereof comprises a FR derived from a human germline light chain IGKV3-11*01 or a FR having at least 95% sequence identity thereto; preferably, the FR comprises a back mutation selected from one or more of 46P, 47W and 71Y; and/or
the anti-IL-4R antibody or the antigen-binding fragment thereof comprises a FR derived from a human germline heavy chain IGHV3-48*01 or a FR having at least 95% sequence identity thereto; preferably, the FR comprises a back mutation selected from one or more of 49A, 67S and 93T.

4. The complex of the anti-IL-4R antibody or the antigen-binding fragment thereof according to any one of claims 1-2, wherein:
the anti-IL-4R antibody or the antigen-binding fragment thereof comprises a FR derived from a human germline light chain IGKV2D-29*01 or a FR having at least 95% sequence identity thereto; preferably, the FR comprises a back mutation selected from the group consisting of 4L and/or 58I; and/or
the anti-IL-4R antibody or the antigen-binding fragment thereof comprises a FR derived from a human germline heavy chain IGHV1-2*02 or a FR having at least 95% sequence identity thereto; preferably, the FR comprises a back mutation selected from one or more of 69L, 71I, 73K and 94K.

5. The complex of the anti-IL-4R antibody or the antigen-binding fragment thereof according to any one of claims 1-2, wherein:
the anti-IL-4R antibody or the antigen-binding fragment thereof comprises a heavy chain constant region, which is a heavy chain constant region of human IgG1, IgG2, IgG3 or IgG4, or a variant thereof; and/or
the antigen-binding fragment is a Fab, Fv, scFv, F(ab')2, dsfv or ScdsFv, preferably a scFv.

6. The complex of the anti-IL-4R antibody or the antigen-binding fragment thereof according to claim 5, wherein:
the scFv comprises a heavy chain variable region, a linking peptide and a light chain variable region in sequence from N-terminus to C-terminus, or
the scFv comprises a light chain variable region, a linking peptide and a heavy chain variable region in sequence from N-terminus to C-terminus;
the linking peptide is selected from any one of the following or a combination thereof: (GxS)ₙ, (SxG)ₙ, (GGGGS)ₙ and (G)ₙ, wherein x is any integer from 1-6, and n is any integer from 1-30; or
the linking peptide is selected from any one of the following or a combination thereof: GKSSGSGSESKS, EGKSSGSGSESKEF, GSTSGSGKSSEGKG, GSTSGSGKSSEGSGSTKG, GSTSGSGKPGSGEGSTKG, SRSSG and SGSSC, preferably (GGGGS)₃.

7. The complex of the anti-IL-4R antibody or the antigen-binding fragment thereof according to any one of claims 1-6, comprising any one selected from the group consisting of (I) to (IV):
(I) a heavy chain variable region comprising the amino acid sequence set forth in SEQ ID NO: 43 or an amino acid sequence having at least 90%, 95%, 98% or 99% identity to SEQ ID NO: 43; and
a light chain variable region comprising the amino acid sequence set forth in SEQ ID NO: 37 or an amino acid sequence having at least 90%, 95%, 98% or 99% identity to SEQ ID NO: 37;
(II) a heavy chain variable region comprising the amino acid sequence set forth in SEQ ID NO: 9 or an amino acid sequence having at least 90%, 95%, 98% or 99% identity to SEQ ID NO: 9; and
a light chain variable region comprising the amino acid sequence set forth in SEQ ID NO: 10 or an amino acid sequence having at least 90%, 95%, 98% or 99% identity to SEQ ID NO: 10;
(III) a heavy chain variable region comprising the amino acid sequence set forth in SEQ ID NO: 1 or an amino acid sequence having at least 90%, 95%, 98% or 99% identity to SEQ ID NO: 1; and
a light chain variable region comprising the amino acid sequence set forth in SEQ ID NO: 2 or an amino acid sequence having at least 90%, 95%, 98% or 99% identity to SEQ ID NO: 2;
(IV) a heavy chain variable region comprising the amino acid sequence set forth in SEQ ID NO: 43 or an amino acid sequence having at least 90%, 95%, 98% or 99% identity to SEQ ID NO: 43; and
a light chain variable region comprising the amino acid sequence set forth in SEQ ID NO: 41 or an amino acid sequence having at least 90%, 95%, 98% or 99% identity to SEQ ID NO: 41;
(V) a heavy chain variable region comprising the amino acid sequence set forth in SEQ ID NO: 47 or an amino acid sequence having at least 90%, 95%, 98% or 99% identity to SEQ ID NO: 47; and
a light chain variable region comprising the amino acid sequence set forth in SEQ ID NO: 48 or an amino acid sequence having at least 90%, 95%, 98% or 99% identity to SEQ ID NO: 48;
preferably,
the heavy chain variable region comprises the sequence set forth in SEQ ID NO: 43, and
the light chain variable region comprises the sequence set forth in SEQ ID NO: 37; or
the heavy chain variable region comprises the sequence set forth in SEQ ID NO: 9, and
the light chain variable region comprises the sequence set forth in SEQ ID NO: 10; or
the heavy chain variable region comprises the sequence set forth in SEQ ID NO: 1, and
the light chain variable region comprises the sequence set forth in SEQ ID NO: 2; or
the heavy chain variable region comprises the sequence set forth in SEQ ID NO: 43, and
the light chain variable region comprises the sequence set forth in SEQ ID NO: 41; or
the heavy chain variable region comprises the sequence set forth in SEQ ID NO: 47, and
the light chain variable region comprises the sequence set forth in SEQ ID NO: 48.

8. The complex of the anti-IL-4R antibody or the antigen-binding fragment thereof according to any one of claims 1-6, wherein:
the heavy chain variable region comprises the amino acid sequence set forth in any one of SEQ ID NOs: 25-27 or an amino acid sequence having at least 90%, 95%, 98% or 99% identity to any one of SEQ ID NOs: 25-27;
the light chain variable region comprises the amino acid sequence set forth in any one of SEQ ID NOs: 28-30 or an amino acid sequence having at least 90%, 95%, 98% or 99% identity to any one of SEQ ID NOs: 28-30;
or
the heavy chain variable region comprises the amino acid sequence set forth in any one of SEQ ID NOs: 31-33 or an amino acid sequence having at least 90%, 95%, 98% or 99% identity to any one of SEQ ID NOs: 31-33;
the light chain variable region comprises the amino acid sequence set forth in any one of SEQ ID NOs: 34-36 or an amino acid sequence having at least 90%, 95%, 98% or 99% identity to any one of SEQ ID NOs: 34-36;
preferably,
the heavy chain variable region comprises the sequence set forth in any one of SEQ ID NOs: 25-27, and the light chain variable region comprises the sequence set forth in any one of SEQ ID NOs: 28-30; or
the heavy chain variable region comprises the sequence set forth in any one of SEQ ID NOs: 31-33, and the light chain variable region comprises the sequence set forth in any one of SEQ ID NOs: 34-36;
more preferably, the heavy chain variable region and/or the light chain variable region comprise(s) a mutation for structural stabilization;
further preferably, the amino acid residue at position 44 of the heavy chain variable region is mutated into a cysteine residue;
further preferably, the amino acid residue at position 100 of the light chain variable region is mutated into a cysteine residue.

9. The complex of the anti-IL-4R antibody or the antigen-binding fragment thereof according to any one of claims 1-6, wherein the anti-IL-4R antibody or the antigen-binding fragment thereof comprises any one selected from the group consisting of (I) to (IV):
(I) a heavy chain comprising the amino acid sequence set forth in SEQ ID NO: 44 or an amino acid sequence having at least 90%, 95%, 98% or 99% identity to SEQ ID NO: 44; and
a light chain comprising the amino acid sequence set forth in SEQ ID NO: 45 or an amino acid sequence having at least 90%, 95%, 98% or 99% identity to SEQ ID NO: 45;
(II) a heavy chain comprising the amino acid sequence set forth in SEQ ID NO: 19 or an amino acid sequence having at least 90%, 95%, 98% or 99% identity to SEQ ID NO: 19; and
a light chain comprising the amino acid sequence set forth in SEQ ID NO: 20 or an amino acid sequence having at least 90%, 95%, 98% or 99% identity to SEQ ID NO: 20;
(III) a heavy chain comprising the amino acid sequence set forth in SEQ ID NO: 17 or an amino acid sequence having at least 90%, 95%, 98% or 99% identity to SEQ ID NO: 17; and
a light chain comprising the amino acid sequence set forth in SEQ ID NO: 18 or an amino acid sequence having at least 90%, 95%, 98% or 99% identity to SEQ ID NO: 18;
(IV) a heavy chain comprising the amino acid sequence set forth in SEQ ID NO: 44 or an amino acid sequence having at least 90%, 95%, 98% or 99% identity to SEQ ID NO: 44; and
a light chain comprising the amino acid sequence set forth in SEQ ID NO: 46 or an amino acid sequence having at least 90%, 95%, 98% or 99% identity to SEQ ID NO: 46; preferably,
the heavy chain comprises the sequence set forth in SEQ ID NO: 44, and the light chain comprises the sequence set forth in SEQ ID NO: 45; or
the heavy chain comprises the sequence set forth in SEQ ID NO: 19, and the light chain comprises the sequence set forth in SEQ ID NO: 20; or
the heavy chain comprises the sequence set forth in SEQ ID NO: 17, and the light chain comprises the sequence set forth in SEQ ID NO: 18; or
the heavy chain comprises the sequence set forth in SEQ ID NO: 44, and the light chain comprises the sequence set forth in SEQ ID NO: 46.

10. The complex of the anti-IL-4R antibody or the antigen-binding fragment thereof according to any one of claims 1-9, wherein:
the anti-IL-4R antibody or the antigen-binding fragment thereof is linked to the toxin molecules by linkers;
the linkers are proteinaceous or non-proteinaceous linkers;
preferably, the complex is a fusion protein.

11. The complex of the anti-IL-4R antibody or the antigen-binding fragment thereof according to claim 10, wherein:
the linkers are selected from any one of the following or a combination thereof: ASGGPE, VM, AM, AM(G₂₋₄S)ₚAM, ASGCGPE, ASGCCGPE, ASGCGSCPE, ASCGTTGCPE, KASGKKYGCKKGPE and KGGGCAGGPE; preferably ASGCGPE; wherein p is any integer from 1-10.

12. The complex of the anti-IL-4R antibody or the antigen-binding fragment thereof according to any one of claims 1-11, wherein the toxin molecules are selected from any one of the following or a combination thereof: a pore-forming toxin, aerolysin from Aeromonas hydrophila, proaerolysin, bouganin, ricin, pseudomonas exotoxin, cholera toxin and diphtheria toxin.

13. The complex of the anti-IL-4R antibody or the antigen-binding fragment thereof according to claim 12, wherein the toxin is selected from any one of the following or a combination thereof: PE-LR, PE-LO10R456A, PE-T20, PE-T20-KDEL, PE4E, PE40, PE38, PE24, PE25, PE38QQR, PE35, PE38KDEL, PE38DKEL, PE38RDEL and PE38KNEL, preferably, PE38KDEL set forth in SEQ ID NO: 49.

14. The complex of the anti-IL-4R antibody or the antigen-binding fragment thereof according to any one of claims 1-13, comprising the sequence set forth in SEQ ID NO: 52 or SEQ ID NO: 53.

15. A complex of an anti-IL-4R antibody or an antigen-binding fragment thereof, comprising:
- the anti-IL-4R antibody or the antigen-binding fragment thereof, and
- one or more toxin molecules;
wherein the anti-IL-4R antibody or the antigen-binding fragment thereof is covalently or non-covalently linked to the toxin molecules;
wherein,
the toxin molecules are selected from any one of the following or a combination thereof: a pore-forming toxin, aerolysin from Aeromonas hydrophila, proaerolysin, bouganin, ricin, pseudomonas exotoxin, cholera toxin and diphtheria toxin;
preferably, the toxin molecules are selected from any one of the following or a combination thereof: PE-LR, PE-LO10R456A, PE-T20, PE-T20-KDEL, PE4E, PE40, PE38, PE24, PE25, PE38QQR, PE35, PE38KDEL, PE38DKEL, PE38RDEL and PE38KNEL;
more preferably, the toxin molecules are PE38KDEL set forth in SEQ ID NO: 49.

16. A polynucleotide encoding the complex of the anti-IL-4R antibody or the antigen-binding fragment thereof according to any one of claims 1 to 15.

17. A vector comprising the polynucleotide according to claim 16, being a eukaryotic expression vector, a prokaryotic expression vector or a viral vector.

18. A host cell comprising the vector according to claim 17, wherein
preferably, the host cell is a bacterial, yeast or mammalian cell;
more preferably, the host cell is *Escherichia coli, Pichia pastoris,* a Chinese hamster ovary cell or a human embryonic kidney 293 cell.

19. A pharmaceutical composition comprising:
the complex of the anti-IL-4R antibody or the antigen-binding fragment thereof according to any one of claims 1 to 15, and
optionally, a pharmaceutically acceptable excipient, diluent or carrier.

20. A method for treating and/or preventing a cancer or tumor, comprising:
administering to a subject a therapeutically or prophylactically effective amount of the complex of the anti-IL-4R antibody or the antigen-binding fragment thereof according to any one of claims 1 to 15, the polynucleotide according to claim 16 or the pharmaceutical composition according to claim 19,
wherein preferably, the cancer or tumor is selected from any one of the following or a combination thereof: prostate cancer, ovarian cancer, breast cancer, endometrial cancer, multiple myeloma, melanoma, lymphoma, lung cancer, kidney cancer, liver cancer, large intestine cancer (e.g., colon cancer), pancreatic cancer, stomach cancer, leukemia and a central nervous system tumor;
more preferably, the central nervous system tumor is selected from any one of the following or a combination thereof: glioma, glioblastoma, neuroblastoma, astrocytoma, medulloblastoma, craniopharyngioma, ependymoma, pinealoma, hemangioblastoma, acoustic neuroma, oligodendroglioma, hemangioma, meningioma and retinoblastoma.

21. The method according to claim 20, wherein the central nervous system tumor is a glioblastoma;
preferably, the central nervous system tumor is a recurrent or refractory glioblastoma, or an O6-methylguanine-DNA methyltransferase expression positive or negative glioblastoma.

22. A method for preparing a complex of an anti-IL-4R antibody or an antigen-binding fragment thereof, comprising the following steps:
expressing the complex of the anti-IL-4R antibody or the antigen-binding fragment thereof in the host cell according to claim 18, and
isolating the complex of the anti-IL-4R antibody or the antigen-binding fragment thereof from the host cell.
